# EUROPEAN PATENT APPLICATION

(11) **EP 2 009 023 A1**
(43) Date of publication of application: **31.12.2008**
(21) Application number: 07109550.9
(22) Date of filing: 04.06.2007
(51) Int. Cl.: C07K 14/525, A61K 38/19, A61P 11/00

(54) **Novel peptides and their use for the treatment of edema**

(71) Applicant: Rentschler Beteiligungs GmbH, 88471 Laupheim (DE)
(72) Inventor: The designation of the inventor has not yet been filed
(74) Representative: Weiss, Wolfgang

(57) **Abstract**

The invention relates to novel peptides, which can be used for the production of pharmaceutical compositions. These pharmaceutical compositions can be used for the treatment of edema, in particular pulmonary edema.

## Description

### Technical field

The invention relates to novel peptides, which can be used for the production of pharmaceutical compositions. These pharmaceutical compositions can be used for the treatment of edema, in particular pulmonary edema.

### Background Art

Current treatments of edema, especially lung edema or pulmonary edema, include taking immediate measures such as placing the patient in a sitting position, administrating oxygen, applying assisted or mechanical ventilation and applying a drug therapy. The current drug therapies are symptomatic drug therapies and include administration of morphine, which is effective in reducing the patients anxiety, easing breathing and improving blood flow, the administration of nitroglycerin, which reduces the pulmonary blood flow and decreases the volume of fluid entering the overloaded blood vessels, the administration of diuretics, like Furosemide, which promote the elimination of fluids through urination, as well as the administration of ACE (angiotensin-converting-enzyme) inhibitors, which reduce the pressure against which the left ventricle must expel blood. For patients with severe hypertension, a vasodilator may be used.

However, none of the currently employed drug therapies does specifically address the resolution of pulmonary edema in the target organ, namely its resorption in the lung epithelium.

In 1997 it has been published that TNF-α is able to up-regulate the rate of alveolar liquid clearance (ALC), in a model of P. aeruginosa elicited pneumonia in rats (Rezaiguia S. et al., J Clin Invest. 99(2), 325 35, 1997). It has been found that this ALC up-regulating TNF-α activity can be mimicked by a 17 amino acid peptide derived from the cytokine TNF-α (Lucas, R. et al, Science 263, 814-817, 1994). It was designated Tip as it sits literally at the tip of TNF and forms a lectin-like domain. In order to mimick its loop structure which it has within TNF-α, it was circularized via a disulfide bond between its terminal cysteine residues. TIP up-regulated alveolar fluid reabsorption in an in situ mouse lung and in an ex vivo (Elia et al., Am J. respire Crit Care Med, 168(9); 1043-50, 2003) and in vivo (Braun, C., et al., J. Immunol, 175(5), 3402-8, 2005) rat lung model and increased membrane conductance in microvascular endothelial cells (Hribar, M. et al., Eur J Immunol, 29(10), 3105-11, 1999). The amino acids Thr105, GIu107, and Glu110 within the Tip domain of human TNF-A-α were shown to be critical for its lectin-like activity (Lucas et al., 1994). Consequently, replacement of these amino acids resulted in an inactive mutant form of Tip. Scrambled Tip, which has also been proven to be inactive contains the same amino acids as TIP but in a randomized order (Lucas et al., 1994; Hribar et al., 1999).

### Disclosure of Invention

### Technical problem

Currently employed treatments that address the life-threatening pathological condition of pulmonary edema do no exert their effects at the site of action and have limited capabilities in addressing the underlying problem, the excess fluid accumulation in lung tissue.

Therefore, there is a high need for effective treatments that induce pulmonary edema resorption by compounds that stimulate active transepithelial fluid transport in the lung.

### Technical solution

It has now been found that the novel peptides of the invention, which are described in greater details below, have surprising and particularly advantageous properties.

In this respect, the present invention provides non-toxic molecules with the same or a better edema resorption-inducing capacity as peptides in the prior art. More specifically, the present invention provides non-toxic peptides, which can be used to prevent or treat edema.

Moreover, the present invention provides a pharmaceutical composition comprising on or more of the novel peptides of the invention, which induce edema resorption.

Furthermore, it is also the object of the present invention to make available a pharmaceutical combination suited for the treatment of edema.

More specifically, the problem has been solved by the following embodiments.

### Mode(s) for Carrying Out the Invention

The invention therefore provides a peptide having a sequence of 14 to 100 contiguous amino acids comprising the sequence TKPIELGPDEPKAV (SEQ ID NO: 76) wherein one, two or three conservative amino acid substitutions are allowed, which peptide inhibits or reduces the accumulation of excess fluid in tissues. Preferably, the peptide has a sequence of 14 to 50 contiguous amino acids.

More specifically, the invention provides a peptide, which consists of the sequence of SEQ ID NO: 76.

In a further preferred embodiment, the invention provides a peptide, which consists of the sequence CGTKPIELGPDEPKAVC (SEQ ID NO: 2).

"Conservative amino acid substitution" within the meaning of the present invention is to be understood as meaning that the amino acid exchange does not lead to an altered structure and function.

Furthermore, peptides are provided having a sequence of 18 to 94 contiguous amino acids comprising the hexameric sequence TPEGAE (SEQ ID NO: 1) which is present at least three times, which peptide inhibits or reduces the accumulation of excess fluid in tissues. Peptides, which comprise multiple copies of the hexameric sequence of the TIP region of the TNF alpha, which has been ascribed a lectin binding activity, are particularly useful in the treatment of edema. Preferred are the novel peptides comprising the hexameric sequence three to five times.

In a further preferred aspect of the present invention, peptides are provided having a sequence of 18 to 94 contiguous amino acids comprising the hexameric sequence TPEGAE (SEQ ID NO: 1), which is present at least three times, which peptide inhibits or reduces the accumulation of excess fluid in tissues, and wherein the hexameric sequence is present as consecutive sequences.

Preferred peptides are the peptides with SEQ ID NO: 3, which do comprise the hexameric sequence three times.

Further preferred peptides are the peptides with SEQ ID NO: 4, which comprise the hexameric sequence four times. Even further preferred peptides are the peptides with SEQ ID NO: 5, which comprise the hexameric sequence five times.

"Consecutive sequences" within the meaning of the present invention is to be understood as meaning that the amino acid sequences are linked to one another via amide bond of the backbone, and are not separated by additional amino acids.

In a further preferred aspect of the present invention, peptides are provided having a sequence of 18 to 94 contiguous amino acids comprising the hexameric sequence TPEGAE which is present at least three times, which peptide inhibits or reduces the accumulation of excess fluid in tissues, and wherein the hexameric sequence is separated by a linker sequence consisting of two to ten amino acids. "Linker sequence" within the meaning of the present invention is to be understood as meaning amino acids present within the amino acid sequence of a peptide of the invention and separating the specific hexameric sequences or the other specifically defined sequences within the peptide. These linker sequences provide for a more flexible conformation of the peptide and positioning of the hexameric sequences or the other specifically defined sequences to each other in the tertiary conformation of the peptide. Furthermore, the linker sequence may also be present at the N- or C-terminus of the peptide, thereby separating the specific hexameric or other specifically defined sequences if the peptide is circularized via the peptide backbone or via N- or C-terminal C amino acid residues.

In a preferred embodiment, the linker sequence consists of two, three, four or five amino acids. In an even more preferred embodiment, the linker sequence consists of five amino acids. Preferably, the linker consists of small amino acid residues. More preferred, the linker sequence consists of one or more of the amino acids selected from the group consisting of G, A, S, P and C. Most preferred, the linker sequence consists of G amino acid residues.

Preferred peptides are the peptides with SEQ ID NO: 9, which do comprise the hexameric sequence three times with a linker sequence of three G residues.

Further preferred peptides are the peptides with SEQ ID NO: 10, which comprise the hexameric sequence three times with a linker sequence of four G residues. Even further preferred peptides are the peptides with SEQ ID NO: 11, which comprise the hexameric sequence three times with a linker sequence of five G residues.

In a preferred embodiment of the present invention, the linker sequences present in the peptides of the present invention are selected from the group consisting of the amino acid sequences GGGGG (SEQ ID NO: 15), GGCGG (SEQ ID NO: 16) and GGSPS (SEQ ID NO: 17).

In a further aspect of the present invention, a peptide is provided having a sequence of 42 to 94 contiguous amino acids comprising the sequence QRETPEGAEAKPWY (SEQ ID NO: 18), which is present at least three times, which peptide inhibits or reduces the accumulation of excess fluid in tissues. Preferred are the peptides comprising the sequence three to five times. Most preferred peptides do comprise the sequence three times.

In a more preferred aspect of the present invention, a peptide is provided having a sequence of 42 to 94 contiguous amino acids comprising the sequence QRETPEGAEAKPWY which is present at least three times, which peptide inhibits or reduces the accumulation of excess fluid in tissues, and wherein the hexameric sequence is present as consecutive sequences.

Therefore, a preferred peptide of the present invention is a peptide of SEQ ID NO: 19.

In a further preferred aspect of the present invention, a peptide is provided having a sequence of 42 to 94 contiguous amino acids comprising the sequence QRETPEGAEAKPWY which is present at least three times, which peptide inhibits or reduces the accumulation of excess fluid in tissues, and wherein the hexameric sequence is separated by a linker sequence consisting of three to ten amino acids.

Therefore, a preferred peptide of the present invention is a peptide of SEQ ID NO: 20.

In another aspect of the present invention, a peptide is provided having a sequence of 6 to 100 contiguous amino acids comprising the hexameric sequence X₁PX₂GX₃X₄ at least one time, wherein X₁ may be selected from S, T and L, wherein X₂ may be selected from D, E, Q and R, wherein X₃ may be selected from A, S, T, E and G, and wherein X₄ may be selected from D, E and K, with the proviso that the combination of amino acids X₁, X₂, and X₄ is not T, E, E, which peptide inhibits or reduces the accumulation of excess fluid in tissues. More specifically, the hexameric sequence is selected from the group of sequences consisting of S**P**E**G**AE (SEQ ID NO: 21); S**P**E**G**GE (SEQ ID NO: 22); S**P**E**G**AD (SEQ ID NO: 23); S**P**E**G**GD (SEQ ID NO: 24); S**P**D**G**AE (SEQ ID NO: 25); S**P**D**G**AD (SEQ ID NO: 26); S**P**D**G**GD (SEQ ID NO: 27); S**P**D**G**GE (SEQ ID NO: 28); T**P**D**G**AE (SEQ ID NO: 29); T**P**D**G**AD (SEQ ID NO: 30); T**P**D**G**GE (SEQ ID NO: 31); T**P**D**G**GD (SEQ ID NO: 32); T**P**E**G**AD (SEQ ID NO: 33); and T**P**E**G**GD (SEQ ID NO: 34).

In a preferred embodiment, the hexameric sequence X₁PX₂GX₃X₄ is present within the peptide from two to eight times. In a more preferred embodiment, the hexameric sequence X₁PX₂GX₃X₄ is present within the peptide from three to five times.

In a more preferred aspect of the invention, a peptide is provided having a sequence of 6 to 100 contiguous amino acids comprising the hexameric sequence X₁PX₂GX₃X₄ at least one time, wherein X₁ may be selected from S, T and L, wherein X₂ may be selected from D, E, Q and R, wherein X₃ may be selected from A, S, T, E and G, and wherein X₄ may be selected from D, E and K, with the proviso that the combination of amino acids X₁, X₂, and X₄ is not T, E, E, which peptide inhibits or reduces the accumulation of excess fluid in tissues, and wherein the hexameric sequence is present as consecutive sequences.

In a further preferred aspect of the invention, a peptide is provided having a sequence of 6 to 100 contiguous amino acids comprising the hexameric sequence X₁PX₂GX₃X₄ at least one time, wherein X₁ may be selected from S, T and L, wherein X₂ may be selected from D, E, Q and R, wherein X₃ may be selected from A, S, T, E and G, and wherein X₄ may be selected from D, E and K, with the proviso that the combination of amino acids X₁, X₂, and X₄ is not T, E, E, which peptide inhibits or reduces the accumulation of excess fluid in tissues, and wherein the hexameric sequence is separated by a linker sequence consisting of three to ten amino acids.

In a further embodiment of the present invention, a peptide is provided having a sequence of 14 to 100 contiguous amino acids comprising the sequence QREX₁PX₂GX₃X₄AKPWY at least one time, wherein X₁ is selected from S, T and L, wherein X₂ is selected from D, E, Q and R, wherein X₃ is selected from A, S, T, E and G, and wherein X₄ is selected from D, E and K, with the proviso that the combination of amino acids X₁, X₂, and X₄ is not T, E, E, which peptide inhibits or reduces the accumulation of excess fluid in tissues.

In a more preferred aspect of the invention, a peptide is provided having a sequence of 14 to 100 contiguous amino acids comprising the sequence QREX₁PX₂GX₃X₄AKPWY at least one time, wherein X₁ is selected from S, T and L, wherein X₂ is selected from D, E, Q and R, wherein X₃ is selected from A, S, T, E and G, and wherein X₄ is selected from D, E and K, with the proviso that the combination of amino acids X₁, X₂, and X₄ is not T, E, E, which peptide inhibits or reduces the accumulation of excess fluid in tissues, and wherein the sequence QREX₁PX₂GX₃X₄AKPWY is present within the peptide as consecutive sequences.

In a further preferred aspect of the invention, a peptide is provided having a sequence of 14 to 100 contiguous amino acids comprising the sequence QREX₁PX₂GX₃X₄AKPWY at least one time, wherein X₁ is selected from S, T and L, wherein X₂ is selected from D, E, Q and R, wherein X₃ is selected from A, S, T, E and G, and wherein X₄ is selected from D, E and K, with the proviso that the combination of amino acids X₁, X₂, and X₄ is not T, E, E, which peptide inhibits or reduces the accumulation of excess fluid in tissues, and wherein the sequence QREX₁PX₂GX₃X₄AKPWY is separated within the peptide by a linker sequence consisting of three to ten amino acids.

In an even more preferred embodiment, a peptide is provided having a sequence of 14 to 100 contiguous amino acids comprising a sequence selected from the group consisting of
QRESPEGAEAKPWY (SEQ ID NO: 62); QRESPEGADAKPWY (SEQ ID NO: 63); QRESPEGGEAK-PWY (SEQ ID NO: 64); QRESPEGGDAKPWY (SEQ ID NO: 65); QRESPDGAEAKPWY (SEQ ID NO: 66); QRESPDGADAKPWY (SEQ ID NO: 67); QRESPDGGEAKPWY (SEQ ID NO: 68); QRESPDGGDAKPWY (SEQ ID NO: 69); QRETPEGADAKPWY (SEQ ID NO: 70); QRETPEGGDAK-PWY (SEQ ID NO: 71); QRETPDGADAKPWY (SEQ ID NO: 72); QRETPDGAEAKPWY (SEQ ID NO: 73); QRETPDGGDAKPWY (SEQ ID NO: 74); and QRETPDGGEAKPWY (SEQ ID NO: 75), at least one time, which peptide inhibits or reduces the accumulation of excess fluid in tissues.

In an even more preferred embodiment, a peptide is provided having a sequence of 17 to 100 contiguous amino acids comprising the sequence selected from the group consisting of
CGQRESPEGAEAKPWYC (SEQ ID NO: 35); CGQRESPEGADAKPWYC (SEQ ID NO: 36); CGQRESPEGGEAKPWYC (SEQ ID NO: 37); CGQRESPEGGDAKPWYC (SEQ ID NO: 38); CGQRESPDGAEAKPWYC (SEQ ID NO: 39); CGQRESPDGADAKPWYC (SEQ ID NO: 40); CGQRESPDGGEAKPWYC (SEQ ID NO: 41); CGQRESPDGGDAKPWYC (SEQ ID NO: 42); CGQRETPEGADAKPWYC (SEQ ID NO: 43); CGQRETPEGGDAKPWYC (SEQ ID NO: 44); CGQRETPDGADAKPWYC (SEQ ID NO: 45); CGQRETPDGAEAKPWYC (SEQ ID NO: 46); CGQRETPDGGDAKPWYC (SEQ ID NO: 47); and CGQRETPDGGEAKPWYC (SEQ ID NO: 48), at least one time, which peptide inhibits or reduces the accumulation of excess fluid in tissues.

Preferred peptides of the present invention comprise linker sequences.
Preferred peptides of the present invention are circularized.
In a most preferred embodiment of the present invention, the above-described peptides comprise linker sequences and are circularized.

In a further preferred embodiment of the present invention, a peptide is provided having an amino acid sequence selected from the group consisting of
GGSPSQRETPEGAEAKPWYGGSPSQRETPEGAEAKPWYGGSPSQRETPEGAEAKPWY (SEQ ID NO: 20);
CGQRESPEGAEAKPWYC (SEQ ID NO: 35);
CGQRESPEGADAKPWYC (SEQ ID NO: 36);
CGQRESPEGGEAKPWYC (SEQ ID NO: 37);
CGQRESPEGGDAKPWYC (SEQ ID NO: 38);
CGQRESPDGAEAKPWYC (SEQ ID NO: 39);
CGQRESPDGADAKPWYC (SEQ ID NO: 40);
CGQRESPDGGEAKPWYC (SEQ ID NO: 41);
CGQRESPDGGDAKPWYC (SEQ ID NO: 42);
CGQRETPEGADAKPWYC (SEQ ID NO: 43);
CGQRETPEGGDAKPWYC (SEQ ID NO: 44);
CGQRETPDGADAKPWYC (SEQ ID NO: 45);
CGQRETPDGAEAKPWYC (SEQ ID NO: 46);
CGQRETPDGGDAKPWYC (SEQ ID NO: 47);
CGQRETPDGGEAKPWYC (SEQ ID NO: 48);
TPEGAEGGGGGTPEGAEGGGGGTPEGAE (SEQ ID NO: 11);
CGTPEGAETPEGAETPEGAEGC (SEQ ID NO: 49);
CGGGGGTPEGAEGGGGGTPEGAEGGGGGTPEGAEGC (SEQ ID NO: 50);
TPEGAETPEGAETPEGAETPEGAETPEGAE (SEQ ID NO: 5);
CGTPEGAETPEGAETPEGAETPEGAETPEGAEGC (SEQ ID NO: 51);
SPEGGEGGGGGSPEGGEGGGGGSPEGGE (SEQ ID NO: 52);
CGSPEGGEGGGGGSPEGGEGGGGGSPEGGEGC (SEQ ID NO: 53);
SPEGGDGGGGGSPEGGDGGGGGSPEGGD (SEQ ID NO: 54);
CGSPEGGDGGGGGSPEGGDGGGGGSPEGGDGC (SEQ ID NO: 55);
CGSPEGGESPEGGESPEGGEGC (SEQ ID NO: 56);
CGSPDGGDSPDGGDSPDGGDGC (SEQ ID NO: 57);
SPDGGDGGGGGSPDGGDGGGGGSPDGGD (SEQ ID NO: 58);
CGSPDGGDGGGGGSPDGGDGGGGGSPDGGDGC (SEQ ID NO: 59);
CGSPEGGDSPEGGDSPEGGDGC (SEQ ID NO: 60); and peptides of SEQ ID NOs: 6, 7, 8, 12, 13, 14, 62, 63, 64, 65, 66, 67, 68, 69, 70, 71, 72, 73, 74, and 75.

In a further preferred embodiment of the present invention, the novel above-described peptides of the invention are provided, which are linear peptides.

"Linear peptides" within the meaning of the present invention is to be understood as meaning that the peptides of the present invention are not cyclic or circularized.

In a further preferred embodiment of the present invention, the peptides of SEQ ID NOs: 2, 3, 4, 5, 6, 7, 8, 9, 10, 11, 12, 13, 14, 19, 20, 35, 36, 37, 38, 39, 40, 41, 42, 43, 44, 45, 46, 47, 48, 49, 50, 51, 52, 53, 54, 55, 56, 57, 58, 59, 60, 62, 63, 64, 65, 66, 67, 68, 69, 70, 71, 72, 73, 74, 75, 76 are provided, which are linear peptides.

In a further preferred embodiment of the present invention, the novel above-described peptides of the invention are provided, which are circularized peptides.

"Circularized peptides" within the meaning of the present invention is to be understood as meaning that the peptides of the present invention are either circularized via an amide bond of the peptide backbone or via the side chain of C amino acid residues at the N- and C-terminus of the peptide forming a disulfide bond. Circularization may improve the half-life of the peptides of the invention in vivo and increase their stability against proteolytic degradation, particularly by exopeptidases. Circularized peptides may be prepared by the use of non-specific carbodiimide for their circularization. In WO0248312, backbone circularized peptide antagonists and methods for their preparation are described. Formation of intramolecular disulfide bonds can, for example, be carried out by oxidative cyclization by the action of iodine.

Therefore, in a more specific embodiment of the present invention, a peptide as described above is provided, wherein said peptide is circularized via an amide bond of the peptide backbone. In a further specific embodiment of the present invention, a peptide as described above is provided, wherein said peptide is circularized via the side chain of C amino acid residues at the N- and C-terminus of the peptide forming a disulfide bond. If any one of the peptides of the invention as described does not have a C amino acid residue at its N- and C-terminus, a C amino acid residue has to be added to the respective terminus of the peptide in order to provide the possibility of its circularization via the side chain of C amino acid residues at its N- and C-terminus.

In this regard, the present invention concerns a peptide as described above wherein said circularized peptide is selected from the group consisting of the circularized peptides selected from the group consisting of
GGSPSQRETPEGAEAKPWYGGSPSQRETPEGAEAKPWYGGSPSQRETPEGAEAKPWY (SEQ ID NO: 20);
CGQRESPEGAEAKPWYC (SEQ ID NO: 35);
CGQRESPEGADAKPWYC (SEQ ID NO: 36);
CGQRESPEGGEAKPWYC (SEQ ID NO: 37);
CGQRESPEGGDAKPWYC (SEQ ID NO: 38);
CGQRESPDGAEAKPWYC (SEQ ID NO: 39);
CGQRESPDGADAKPWYC (SEQ ID NO: 40);
CGQRESPDGGEAKPWYC (SEQ ID NO: 41);
CGQRESPDGGDAKPWYC (SEQ ID NO: 42);
CGQRETPEGADAKPWYC (SEQ ID NO: 43);
CGQRETPEGGDAKPWYC (SEQ ID NO: 44);
CGQRETPDGADAKPWYC (SEQ ID NO: 45);
CGQRETPDGAEAKPWYC (SEQ ID NO: 46);
CGQRETPDGGDAKPWYC (SEQ ID NO: 47);
CGQRETPDGGEAKPWYC (SEQ ID NO: 48);
TPEGAEGGGGGTPEGAEGGGGGTPEGAE (SEQ ID NO: 11);
CGTPEGAETPEGAETPEGAEGC (SEQ ID NO: 49);
CGGGGGTPEGAEGGGGGTPEGAEGGGGGTPEGAEGC (SEQ ID NO: 50);
TPEGAETPEGAETPEGAETPEGAETPEGAE (SEQ ID NO: 5);
CGTPEGAETPEGAETPEGAETPEGAETPEGAEGC (SEQ ID NO: 51);
SPEGGEGGGGGSPEGGEGGGGGSPEGGE (SEQ ID NO: 52);
CGSPEGGEGGGGGSPEGGEGGGGGSPEGGEGC (SEQ ID NO: 53);
SPEGGDGGGGGSPEGGDGGGGGSPEGGD (SEQ ID NO: 54);
CGSPEGGDGGGGGSPEGGDGGGGGSPEGGDGC (SEQ ID NO: 55);
CGSPEGGESPEGGESPEGGEGC (SEQ ID NO: 56);
CGSPDGGDSPDGGDSPDGGDGC (SEQ ID NO: 57);
SPDGGDGGGGGSPDGGDGGGGGSPDGGD (SEQ ID NO: 58);
CGSPDGGDGGGGGSPDGGDGGGGGSPDGGDGC (SEQ ID NO: 59);
CGSPEGGDSPEGGDSPEGGDGC (SEQ ID NO: 60); and peptides of SEQ ID NOs: 6, 7, 8, 12, 13, and 14.

In a further preferred embodiment of the present invention, the peptides of SEQ ID NOs: 2, 3, 4, 5, 6, 7, 8, 9, 10, 11, 12, 13, 14, 19, 20, 35, 36, 37, 38, 39, 40, 41, 42, 43, 44, 45, 46, 47, 48, 49, 50, 51, 52, 53, 54, 55, 56, 57, 58, 59, 60, 62, 63, 64, 65, 66, 67, 68, 69, 70, 71, 72, 73, 74, 75, 76 are provided, which are circularized via an amide bond of the peptide backbone.

In a further preferred embodiment of the present invention, the peptides of SEQ ID NOs: 2, 3, 4, 5, 6, 7, 8, 9, 10, 11, 12, 13, 14, 19, 20, 35, 36, 37, 38, 39, 40, 41, 42, 43, 44, 45, 46, 47, 48, 49, 50, 51, 52, 53, 54, 55, 56, 57, 58, 59, 60, 62, 63, 64, 65, 66, 67, 68, 69, 70, 71, 72, 73, 74, 75, 76 are provided, which are circularized peptides via the side chain of a respective C amino acid residue at the N- and C-terminus of the peptide forming an intramolecular disulfide bond.

In an even further aspect of the present invention, the intramolecular disulfide bonds are formed within the peptide sequence via the side chains of C residues within the linker sequence.

In a further preferred embodiment of the present invention, the herein before mentioned peptides are provided which are synthetic peptides.

"Synthetic peptides" within the meaning of the present invention is to be understood as meaning that the peptides of the present invention are prepared by chemical synthesis. The amino acids used in this invention are those, which are available commercially or are available by routine synthetic methods. Certain residues may require special methods for incorporation into the peptide, and either sequential, divergent and convergent synthetic approaches to the peptide sequence are useful in this invention. Natural coded amino acids and their derivatives are represented by one-letter codes according to IU-PAC conventions. When there is no indication, the L isomer of the amino acid was used.

The peptides of the present invention can be prepared by any method known in the art such as classical chemical synthesis, as described by Houben-Weyl (Houben-Weyl Methods of Organic Chemistry. Synthesis of Peptides and Peptidomimetics, M. Goodman, A. Felix, L. Moroder, C. Toniolo, Eds), Georg Thieme Verlag, Stuttgart and New York, 2003). Chemical synthesis of peptides is well known in the art. Solid phase synthesis is commonly used and various commercial synthesizers are available, for example automated synthesizers by Applied Biosystems Inc., Foster City, CA; Beckman; Multi-Syntech, Bochum, Germany etc. Solution phase synthetic methods may also be used, although it is less convenient. By using these standard techniques, naturally occurring amino acids may be substituted with unnatural amino acids, particularly D-stereoisomers, and also with amino acids with side chains having different lengths or functionalities. Functional groups for conjugating to small molecules, label moieties, peptides, or proteins, or for purposes of forming circularized peptides may be introduced into the molecule during chemical synthesis. In addition, small molecules and label moieties may be attached during the synthetic process. Preferably, introduction of the functional groups and conjugation to other molecules minimally affects the structure and function of the subject peptide.

The N-and C-terminus may be derivatized using conventional chemical synthetic methods. The peptides of the invention may contain an acyl group, such as an acetyl group.

Methods for acylating, and specifically for acetylating the free amino group at the N-terminus are well known in the art. For the C-terminus, the carboxyl group may be modified by esterification with alcohols or amidated to form-CONH2 or CONHR. Methods of esterification and amidation are done using well-known techniques.

In a further preferred of the present invention, the herein before mentioned peptides are provided which are produced recombinantly. "Recombinant production" within the meaning of the present invention is to be understood as meaning that the peptides of the present invention are prepared by means of recom-binant DNA techniques as described in Molecular Cloning (Molecular Cloning: A Laboratory Manual, by Joseph Sambrook and David Russell, Third Edition, Cold Spring Harbor Laboratory, 2001, 3 Volumes) and, more specifically, by Lucas et al. (Lucas, R. et al, Science 263, 814-817, 1994).

It should be clear that the present invention does not exclude post- translational modifications of the peptides such as glycosylation, acetylation, phosphorylation, modifications with fatty acids and the like. Included within present invention are, for example, peptides containing one or more analogues of an amino acid (including unnatural amino acids), peptides with substituted linkages, peptides containing disulfide bonds between cysteine residues, as well as other modifications known in the art.

The identity and activity of the peptides and their mixtures can be determined by methods known in the art. The identity of the peptides can be investigated by amino acid sequencing.

The properties of the peptides of the invention can be investigated by assays and methods as known in the art and those as described below. The property of the peptides of the invention not having a pro-inflammatory effect, e.g. via the TNF-α receptor, for example, can also be tested by in-vivo methods as decribed below. Additionally, the peptides of the invention can be evaluated in standard assay for toxicity.

Patch clamp studies with A549 cells, murine microvascular endothelial cells and peritoneal macrophages can be used to show that compounds induce an amiloride-sensitive current indicating the activation of sodium specific ion channels (Fukuda et al., Am. J Physiol Lung Cell Mol Physiol, 280 (6), 1258-65, 2001; Hribar et al., 1999). However, this technique turned out to be not suitable as a screening tool for anti-pulmonary edema drug candidates with respect to statistical reliability. Therefore, a stable and statistically reliable in vitro test system is needed to assess the activity of anti-pulmonary edema compounds that directly activate active fluid transport in the lung epithelium. For this purpose the ability of the human pulmonary epithelial cell line Calu-3 to spontaneously form domes within confluent monolayers grown on a nonporous substrate (dome assay) can be used. Domes are fluid filled mounds of cells that result from active ion transport from the apical to the basal surface with water following passively and can therefore be used to mimic the process of active fluid resorption in vitro (Mason, R. J., et al., Proc.Natl.Acad.Sci.U.S.A, 79(19):6033-7,1982; Goodman, B. E. and Crandall, E. D., Am. J. Physiol. 2(1) C96-100, 1982; Goodman, B. E., et al., Am. J Physiol, 245(1):C78-C83, 1983).
In a complementary approach the effect of the novel peptides of the invention on bioelectric properties of polarized Calu-3 cell monolayers (Transepithelial electrical resistance (TEER) assay) can be examined to assess the influence of the peptides on transepithelial ion currents.
Two different in vitro test systems, using the lung epithelial Calu 3 cells, allow the screening and identification of anti-lung edema drug candidates. (1) The ability of Calu-3 cells for spontaneous dome formation within confluent monolayers allows quantitative examination of transepithelial fluid transport ("dome assay"). With dome assay experiments it can be confirmed that dome formation by lung epithelial cells is a sodium dependent process and that the peptides of the invention are able to increase this process. (2) Bioelectrical properties of polarized Calu-3 cell monolayers can be studied in a Transepithelial Electrical Resistance (TEER) assay. With the TEER assay experiments it can be shown that the peptides of the invention influence transepithelial conductance of polarized Calu-3 cell monolayers in a polarized and dose dependent manner.

For dome assay experiments cells are seeded into 96-well plates at a density of 5 x 104 cells per well in a volume of 200 µl and cultured until they reach full confluency and start to form domes. Prior to the addition of the peptides of the invention, dome density is documented by microscopic photography of each well with the optical image analysis system CellScreen (Innovatis, Bielefeld, Germany) and analyzed with the CS Processing software package (Innovatis). After addition of compounds dome density is recorded every hour for up to 4 h. After completion of an experiment, image data of each well are analyzed by counting domes of a defined area of 12.5 mm². Data are presented as percentage of domes compared with the dome density before compound addition.
In the ion-substitution experiments the culture medium is removed by aspiration and the cell monolayers are incubated with NMDG-buffer. In control wells medium is replaced by NaCl-buffer.

Several in vivo models can be used to investigate the activity and the properties of the novel pe ptides of the invention.

Proof of concept animal studies can be performed using different species and different lung edema models, including permeability and hydrostatic lung edema models. They fall into two classes: First, the intact lung models and secondly, the injured lung and hydrostatic lung edema models. Examples of each (a) intact lung and (b) injured lung and hydrostatic edema animal models are the following:

In the "In situ flooded rat lung model", rats are anesthetized, intubated and lungs inflated in inspiratory hold while the animal was exsanguinated. In the dead animal, the lung remains in situ. Saline + 2% albumine +/- drug compound are instilled intratracheally. After 30 min, lung fluid samples are collected and the albumine concentration is determined and used for calculation of alveolar liquid clearance (ALC) (Braun, C., et al., J. Immunol, 175(5), 3402-8, 2005; Rezaiguia S. et al., J Clin Invest. 99(2), 325 35, 1997).

In the "In vivo flooded rat lung model", the rats are anaesthetized, intubated and ventilated and kept stable (monitored by blood pressure) for 40-50 min. Then the test compounds (in pre-warmed saline) are instilled intratracheally. The endpoints are arterial blood gases which are recorded for 80 min; and at the end, lungs are excised and the wet to dry ratio measured (Braun, C., et al., J. Immunol, 175(5), 3402-8, 2005).

In the "Endotoxin induced lung injury in the isolated blood perfused flooded rat lung model", which is an ex vivo permeability lung edema model, E. coli-LPS is instilled intratracheally and after approximately 5 hours the lung is isolated and placed in an artificial thorax chamber. Then, perfusion occurs with autolo-gous blood. After an one hour stabilisation period, the test compound (in 1 ml saline) is instilled intratra-cheally. Lung parameters are measured over 2 hours. The main read-outs are lung-weight and parame-ters of lung mechanics and hemodynamics.

In the "Isolated perfused rabbit lung", two sorts of proof of concept studies with inhaled test compounds can be performed: A hydrostatic challenge model and a combined endo/exotoxin lung injury model. The main read-outs are the capillary filtration coefficient, which describes the liquid flux between the vessels and the interstitium, and lung weight as a surrogate parameter for retention. The capillary filtration coef-ficient is assessed by hydrostatic challenges via increasing PAP (= pulmonary artery pressure).

In a further aspect of the present invention, salts the herein before mentioned peptides are provided. "Salts" of the peptide of the present invention are physiologically acceptable organic or inorganic salts, comprising acetate salts of the peptides of the present invention.

In a further aspect of the present invention, a peptide mixture is provided containing at least one of the beforehand-described peptides of the invention. The peptide mixture may therefore comprise two, three, four, five, up to ten or more of the peptides selected from the group consisting of SEQ ID NOs: 2, 3, 4, 5, 6, 7, 8, 9, 10, 11, 12, 13, 14, 19, 20, 35, 36, 37, 38, 39, 40, 41, 42, 43, 44, 45, 46, 47, 48, 49, 50, 51, 52, 53, 54, 55, 56, 57, 58, 59, 60, 62, 63, 64, 65, 66, 67, 68, 69, 70, 71, 72, 73, 74, 75, and 76.

The present invention further provides a peptide or a peptide mixture of the present invention as described beforehand or a pharmaceutically acceptable salt thereof for use in the treatment and/or prophylaxis of diseases.

The present invention furthermore relates to a pharmaceutical composition comprising one or more peptides of the present invention as described beforehand, or a pharmaceutically acceptable salt thereof together with one or more pharmaceutically acceptable auxiliaries and/or excipients.

Suitable auxiliaries or excipients known to the skilled man are saline, Ringer's solution, dextrose solution, Hank's solution, fixed oils, ethyl oleate, 5% dextrose in saline, substances that enhance isotonicity and chemical stability, buffers and preservatives. Other suitable carriers include any carrierthat does not itself induce the production of antibodies harmful to the individual receiving the composition such as proteins, polysaccharides, polylactic acids, polyglycolic acids, polymeric amino acids and amino acid copolymers. The "medicament" may be administered by any suitable method within the knowledge of the skilled man. The preferred route of administration is parenterally. In parenteral administration, the medicament of this invention will be formulated in a unit dosage injectable form such as a solution, suspension or emulsion, in association with the pharmaceutically acceptable excipients as defined above. However, the dosage and mode of administration will depend on the individual. Generally, the medicament is administered so that the peptide of the present invention is given at a dose between 1 pg/kg and 10 mg/kg, more preferably between 10 pg/kg and 5 mg/kg, most preferably between 0.1 and 2 mg/kg. Preferably, it is given as a bolus dose. Continuous infusion may also be used. If so, the medicament may be infused at a dose between 5 and 20 pg/kg/minute, more preferably between 7 and 15 pg/kg/minute.

### Combinations:

In another aspect of present invention there is provided a pharmaceutical composition comprising an effective amount of a pulmonary surfactant and an effective amount of one of the herein before mentioned peptides.

In another aspect of present invention there is provided a pharmaceutical composition comprising an effective amount of a pulmonary surfactant and an effective amount of one of the herein before mentioned peptides, wherein the pulmonary surfactant is selected from the group consisting of PORACTANT ALFA, BERACTANT, BOVACTANT, COLFOSCERIL PALMITATE, SURFACTANT-TA, CALFACTANT, PUMACTANT, LUSUPULTIDE and SINAPULTIDE.

In another aspect of present invention there is provided a pharmaceutical composition comprising an effective amount of a pulmonary surfactant and an effective amount of one of the herein before mentioned peptides, wherein the pulmonary surfactant is selected from the group consisting of PORACTANT ALFA, BERACTANT, BOVACTANT, COLFOSCERIL PALMITATE, SURFACTANT-TA, CALFACTANT, PUMACTANT, LUSUPULTIDE and SINAPULTIDE AND WHEREIN the peptide is selected from the group consisting of the peptides of SEQ ID NOs: 2, 3, 4, 5, 6, 7, 8, 9, 10, 11, 12, 13, 14, 19, 20, 35, 36, 37, 38, 39, 40, 41, 42, 43, 44, 45, 46, 47, 48, 49, 50, 51, 52, 53, 54, 55, 56, 57, 58, 59, 60, 62, 63, 64, 65, 66, 67, 68, 69, 70, 71, 72, 73, 74, 75, 76.

In another aspect of present invention there is provided a pharmaceutical composition comprising an effective amount of a pulmonary surfactant and an effective amount of one of the herein before mentioned peptides for use as a medicament.

In another aspect of present invention there is provided a fixed combination of (1) an effective amount of a pulmonary surfactant and (2) an effective amount of one of the herein before mentioned peptides, and (3) optionally a pharmaceutically acceptable carrier.

In another aspect of present invention there is provided a pharmaceutical composition in powder form comprising as a fixed combination of (1) an effective amount of a pulmonary surfactant and (2) an effective amount of one of the herein before mentioned peptides, and (3) optionally a pharmaceutically acceptable carrier.

In another aspect of present invention there is provided a pharmaceutical composition in liquid form comprising as a fixed combination of (1) an effective amount of a pulmonary surfactant and (2) an effective amount of one of the herein before mentioned peptides, and (3) optionally a pharmaceutically acceptable carrier.

In another aspect of present invention there is provided a pharmaceutical composition for intratra-cheally or intrabronchially instillation comprising as a fixed combination (1) an effective amount of a pulmonary surfactant and (2) an effective amount of one of the herein before mentioned peptides, and (3) optionally a pharmaceutically acceptable carrier.

In another aspect of present invention there is provided a pharmaceutical composition for inhalation comprising as a fixed combination (1) an effective amount of a pulmonary surfactant and (2) an effective amount of one of the herein before mentioned peptides, and (3) optionally a pharmaceutically acceptable carrier.

In another aspect of present invention there is provided a free combination of (1) an effective amount of a pulmonary surfactant and optionally a pharmaceutically acceptable carrier and (2) an effective amount of one of the herein before mentioned peptides and optionally a pharmaceutically acceptable carrier.

In another aspect of present invention there is provided a pharmaceutical composition in liquid form comprising as a free combination (1) an effective amount of a pulmonary surfactant and optionally a pharmaceutically acceptable carrier and (2) an effective amount of one of the herein before mentioned peptides and optionally a pharmaceutically acceptable carrier.

In another aspect of present invention there is provided a pharmaceutical composition in powder form comprising as a free combination (1) an effective amount of a pulmonary surfactant and optionally a pharmaceutically acceptable carrier and (2) an effective amount of one of the herein before mentioned peptides and optionally a pharmaceutically acceptable carrier.

In another aspect of present invention there is provided a pharmaceutical composition for intratra-cheally or intrabronchially instillation comprising as a free combination (1) an effective amount of a pulmonary surfactant and optionally a pharmaceutically acceptable carrier and (2) an effective amount of one of the herein before mentioned peptides and optionally a pharmaceutically acceptable carrier.

The "pulmonary surfactant" useful in this invention may be any compound or pulmonary surfactant preparation that is known to have the same surface-active properties as natural pulmonary surfactant; natural pulmonary surfactant reduces, for example, the surface tension in the alveoli.

A simple and rapid in vitro test with which the surface activity of pulmonary surfactant can be determined is, for example, the so-called Wilhelmy balance (Goerke, J. Biochim. Biophys. Acta, 344: 241-261 (1974), King R.J. and Clements J.A., Am. J. Physicol. 223: 715-726, 1972). This method gives information on the pulmonary surfactant quality, measured as the action of a pulmonary surfactant of achieving a surface tension of almost zero mN/m. Another measuring device for determining the surface activity of pulmonary surfactant is the pulsating bubble surfactometer (Possmayer F. et al., Prog. Resp. Res., Ed. v. Wichert, Vol. 18: 112-120, 1984). The activity of a pulmonary surfactant preparation can also be determined by means of in vivo tests, for example as described by Häfner et al. (D. Häfner et al.: Effects of rSP-C surfactant on oxygenation and histology in a rat lung lavage model of acute lung injury. Am. J. Respir. Crit. Care Med. 1998, 158: 270 278).

A group of known pulmonary surfactant preparations and their modifications that may be usefully employed as pulmonary surfactant in the present invention include pulmonary surfactant preparations having the function of natural pulmonary surfactant. Preferred pulmonary surfactant preparations are those, which, for example, have activity in the tests described above. Particularly preferred pulmonary surfactant preparations are those, which exhibit increased activity in such a test in comparison with natural, in particular human, pulmonary surfactant. In this context, these pulmonary surfactant preparations may be compositions, which only contain phospholipids, but may also be compositions, which, apart from the phospholipids, inter alia additionally contain pulmonary surfactant protein.

Preferred phospholipids according to the invention are dipalmitoylphosphatidylcholine (DPPC), palmitoyloleylphosphatidylglycerol (POPG) and/or phosphatidylglycerol (PG). Particularly preferably, the phospholipids are mixtures of various phospholipids, in particular mixtures of dipalmitoylphosphatidylcholine (DPPC) and palmitoyloleylphosphatidylglycerol (POPG), preferably in the ratio from 7 to 3 to 3 to 7.

Commercial products, which may be mentioned as pulmonary surfactant preparations are
■ CUROSURF® (INN: PORACTANT ALFA) (Serono, Pharma GmbH, Unterschleißheim), a natural surfactant from homogenized porcine lungs;
■ SURVANTA® (INN: BERACTANT) (Abbott GmbH, Wiesbaden), extract of bovine lungs;
■ ALVEOFACT® (INN: BOVACTANT) (Boehringer Ingelheim), extract of bovine lungs;
■ EXOSURF® (INN: COLFOSCERIL PALMITATE) (Glaxo SmithKline), a synthetic phospholipid containing excipients;
■ SURFACTEN® (INN: SURFACTANT-TA) (Mitsubishi Pharma Corporation), a pulmonary surfactant extracted from bovine lungs;
■ INFASURF® (INN: CALFACTANT) (Forest Pharmaceuticals), a surfactant extracted from calf lungs;
■ ALEC® (INN: PUMACTANT) (Britannia Pharmaceuticals), an artificial surfactant of DPPC and PG; and
■ BLES® (BLES Biochemical Inc.), a bovine lipid extract surfactant.

Suitable pulmonary surfactant proteins are both the proteins obtained from natural sources, such as pulmonary lavage or extraction from amniotic fluid, and the proteins prepared by genetic engineering or chemical synthesis. According to the invention, in particular the pulmonary surfactant proteins desig-nated by SP B (Surfactant Protein-B) and SP C (Surfactant Protein-C) and their modified derivatives are of interest. The amino acid sequences of these pulmonary surfactant proteins, their isolation or preparation by genetic engineering are known (e.g. from WO8603408, EP0251449, WO8904326, WO8706943, WO8803170, WO9100871, EP0368823 and EP0348967). Modified derivatives of the pulmonary surfactant proteins designated by SP-C, which differ from human SP C by the replacement of a few amino acids, are described, for example, in WO9118015 and WO9532992. Particularly to be emphasized in this connection are the recombinant SP-C (rSP-C) derivatives which are disclosed in WO9532992, in particular those which differ from human SP C in positions 4 and 5 by the substitution of cysteine by phenylalanine and in position 32 by the substitution of methionine by isoleucine (designated herein as rSP-C (FF/I) or LUSUPULTIDE (INN) or VENTICUTEâ). Modified derivatives of pulmonary surfactant proteins are also understood as meaning those proteins which have a completely originally designed amino acid sequence with respect to their pulmonary surfactant properties, such as are described in EP0593094 and WO9222315. Preferably, the polypeptide KL4 (INN: SINAPULTIDE, SURFAXINâ) may be mentioned in this connection. The name pulmonary surfactant protein, according to the invention, also comprises mixtures of different pulmonary surfactant proteins. In EP0100910, EP0110498, EP0119056, EP0145005 and EP0286011 phospholipid compositions with and without pulmonary surfactant proteins are described which are likewise suitable as components of the prepara-tions.
As a further constituent, which can be present in pulmonary surfactant preparations, fatty acids such as palmitic acid may be mentioned. The pulmonary surfactant preparations can also contain electrolytes such as calcium, magnesium and/or sodium salts (for example calcium chloride, sodium chloride and/or sodium hydrogencarbonate) in order to establish an advantageous viscosity. Preferred pulmonary surfactant preparations according to the invention contain 80 to 95% by weight of phospholipids, 0.5 to 3.0% by weight of pulmonary surfactant proteins, 3 to 15% by weight of fatty acid, preferably palmitic acid, and 0 to 3% by weight of calcium chloride.

The pulmonary surfactant preparations are prepared by processes known per se and familiar to the person skilled in the art, for example as described in WO9532992. According to the invention, the pulmonary surfactant preparations are preferably lyophilized and in particular spray-dried pulmonary surfactant preparations. Lyophilized preparations are disclosed, for example, in WO9735882, WO9100871 and DE3229179. WO9726863 describes a process for the preparation of powdered pulmonary surfactant preparations by spray drying. According to the invention, preparations prepared in this way are preferred.

The term "carrier" within the meaning of the present invention is to be understood as any pharmaceutically acceptable substance or excipient, such as, for example H₂0, or H₂0 and NaCl, or 0,9% NaCl solution.

The phrase "combined use" (or "combination") embraces the administration of a pulmonary surfactant and a peptide of present invention as part of a specific treatment regimen intended to provide a beneficial effect from the co-action of these therapeutic agents. Administration of these therapeutic agents in combination typically is carried out over a defined time period (usually minutes, hours, days or weeks depending upon the combination selected). "Combined use" generally is not intended to encompass the administration of two of these therapeutic agents as part of separate monotherapy regimens that incidentally and arbitrarily result in the combinations of the present invention.

"Combined use" or "combination" within the meaning of the present invention is to be understood as meaning that the individual components of the combination can be administered simultaneously (in the form of a combination medicament - "fixed combination") or more or less simultaneously, respectively in succession (from separate pack units - "free combination"; directly in succession or else alternatively at a relatively large time interval) in a manner which is known per se and customary. As an example, one therapeutic agent could be taken in the morning and one later in the day. Or in another scenario, one therapeutic agent could be taken once daily and the other twice weekly. It is understood, that if individual components are administered directly in succession, the delay in administering the second component should not be such as to lose the beneficial therapeutic effect of the combination.

It is to be understood that present invention covers all combinations of particular and preferred aspects of the invention described herein. Thus, present invention clearly refers to all compounds or preparations mentioned herein as examples of a pulmonary surfactant and to all compounds mentioned herein as a peptide and to all possible consequential combinations.

In particular, combinations, which may be mentioned as preferred examples of a combination of a pulmonary surfactant and a peptide of the invention are:
Combinations of one of the peptides selected from the group of SEQ ID NOs: 2, 3, 4, 5, 6, 7, 8, 9, 10, 11, 12, 13, 14, 19, 20, 35, 36, 37, 38, 39, 40, 41, 42, 43, 44, 45, 46, 47, 48, 49, 50, 51, 52, 53, 54, 55, 56, 57, 58, 59, 60, 62, 63, 64, 65, 66, 67, 68, 69, 70, 71, 72, 73, 74, 75, 76 and LUSUPULTIDE.
Combinations of two, three, four, five, up to ten, or more of the peptides selected from the group of SEQ ID NOs: 2, 3, 4, 5, 6, 7, 8, 9, 10, 11, 12, 13, 14, 19, 20, 35, 36, 37, 38, 39, 40, 41, 42, 43, 44, 45, 46, 47, 48, 49, 50, 51, 52, 53, 54, 55, 56, 57, 58, 59, 60, 62, 63, 64, 65, 66, 67, 68, 69, 70, 71, 72, 73, 74, 75, 76 and LUSUPULTIDE.

More or less simultaneous administration of each therapeutic agent can be effected by, for example, intratracheal or intrabronchial administration to the subject in need thereof either as an instillation of the dissolved therapeutic agents, or as an aerosolised liquid or as a dry powder having a fixed ratio of each therapeutic agent.

Administration of each therapeutic agent in succession, close in time or remote in time, can be effected by any appropriate route, including, but not limited to, intratracheal or intrabronchial instillation or by inhalation. The therapeutic agents can be administered by the same route or by different routes. For example, a pulmonary surfactant may be administered by intratracheal or intrabroncheal instillation while the peptide may be administered by inhalation. The sequence in which the therapeutic agents are administered is not narrowly critical.

The term "liquid form" within the meaning of the present invention is to be understood as any liquid formulation of the pulmonary surfactant or the peptide, such as, for example, a suspension, or a solution, or, as another example, a solution of the peptide containing the pulmonary surfactant in suspended form.

The most preferred route of administration of a pulmonary surfactant is the intratracheal or intrabronchial route by instillation in liquid form or as aerosolised liquid or as dry powder. It is also preferred that the pulmonary surfactant is administered in form of an aerosolised liquid or a dry powder by inhalation. Dry powder formulations of pulmonary surfactants are preferably prepared by the spray drying process as described in WO9726863.

The most preferred route of administration of a peptide of present invention is the intratracheal or intrabronchial route by instillation in liquid form or as aerosolized or nebulized solution or as dry powder.

In case of intratracheal or intrabronchial administration of a pulmonary surfactant preparation, it has proven advantageous to administer suspensions or solutions of the preparations according to the invention which contain 10 to 100 mg of phospholipids per ml of suspension. Preferably, the preparations according to the invention are administered per application in such an amount that the amount of phospholipids is between 10 and 400 mg per kilogram of body weight. As a rule, administration is carried out 1 to 3 times daily over a period of 1 to 7 days. A process is preferred in which the pulmonary surfactant suspension or solution employed contains 0.5 to 2.0 mg of rSP-C (FF/I) per ml of solvent. Particular mention may be made of a process in which the pulmonary surfactant solution employed contains 0.75 to 1.5 mg of rSP-C (FF/I) per ml of solvent.

Generally, a peptide of present invention is administered at a dose between 1 µg/kg and 10 mg/kg, more preferably between 10 µg/kg and 5 mg/kg, most preferably between 0. 1 and 2 mg/kg. In case of intratracheal or intrabronchial instillation a peptide of present invention is administered at a dose of 1-10 mg/ml/kg body weight, particularly preferred at a dose of 1-2 mg/ml/kg body weight. In case the peptide of present invention is administered as an aerosol or by intratracheal nebulization, the peptide is administered in a dose between 1-10 mg/ml/kg body weight, particularly preferred at a dose of 1-2 mg/ml/kg body weight.

In spite of this, if appropriate it may sometimes be necessary to depart from the amounts mentioned, mainly depending on the body weight or the type of administration route, on individual behavior towards the medicament, the manner of its formulation and the time or interval at which administration takes place. Thus in some cases it may be sufficient to manage with less than the abovementioned minimum amount, while in other cases the upper limit mentioned has to be exceeded. In the case of the administration of relatively large amounts, it may be advisable to divide these into several individual doses over the course of the day.

In case of fixed pharmaceutical compositions, which are intended for intratracheal or intrabronchial instillation or for inhalation, the therapeutic agent(s) are formulated to give medicaments according to processes known per se and familiar to the person skilled in the art. The therapeutic agents are employed as a medicament, preferably in combination with a salt, such as NaCl, preferably in 0,9 % NACI, the therapeutic agent content advantageously being between 0.1 and 95% by weight of total volume.

In case of free pharmaceutical compositions, which are intended for intratracheal or intrabronchial instillation, each therapeutic agent is formulated to give a medicament according to processes known per se and familiar to the person skilled in the art. Each therapeutic agent is employed as a medicament, preferably in combination with a salt, such as NaCl, preferably in 0,9 % NACI, the therapeutic agent content advantageously being between 0.1 and 95% by weight of total volume.

The therapeutic agent(s) of the present invention can be administered by a variety of methods known in the art, although for many therapeutic applications, the preferred route of administration is a fixed combination of a pulmonary surfactant and a peptide of present invention whereby the combination product is administered as a dry powder or as an aerosol by inhalation or by intratracheal or intrabronchial instillation of a liquid. For some therapeutic application it may be preferable to admi nister the pulmonary surfactant and the peptide of present invention as a free combination, whereby the preferred route of administration is the intrabronchial instillation of single liquid form ulations.

The therapeutic agent(s) are dosed in an order of magnitude customary for the individual dose. It is more likely possible that the individual actions of the therapeutic agents are mutually positively influenced and reinforced and thus the respective doses on the combined administration of the therapeutic agent(s) may be reduced compared with the norm.

### Industrial applicability

In a further aspect the present invention relates to the use of a peptide of the present invention as beforehand described or a pharmaceutically acceptable salt thereof in the manufacture of a pharmaceutical composition for the treatment and or prevention of a disease, in which inhibition or reduction of excess fluid accumulation in tissues is beneficial.

"Inhibition or reduction of excess fluid accumulation in tissues" within the meaning of the present invention is to be understood as any inhibition or reduction of such fluid accumulation, which may be achieved by, e.g., the regulation or activation of active transepithelial ion transport resulting in passive water flow over the cell membranes.

In a preferred embodiment of the present invention, the disease is edema.

The term "edema" relates to any abnormal excess accumulation of (serous) fluid in connective tissue or in a serous cavity.

In an even more preferred embodiment of the present invention, the disease is lung or pulmonary edema.

According to this invention, pulmonary edema is characterized by a shift of liquid from the pulmonary vessels to the interstitial spaces and the alveolar lumen (interstitial or alveolar edema). Based on their genesis, edema may be divided in hydrostatic and permeability edema, with hydrostatic edema having cardiogenic origin (high blood pressure) and permeability edema occurring after alterations, which lead to higher permeability of the endothelial and/or epithelial cell layer at the airway/vessel interface in the lung.

Lung or pulmonary edema is a common medical emergency and can even be life-threatening. Fluid accumulation in the interstitial tissue and distal airspaces of the lungs results in an impaired gas exchange leading to an unacceptably low oxygen level in the blood. Patients with pulmonary edema show typical symptoms including shortness of breath, lung-cracking sounds, pink-stained sputum cough, and anxiety. During edema formation the excessive fluid first accumulated in the interstitial spaces of the lung (interstitial pulmonary edema), producing only a few clinical symptoms. At a later stage, flooding of the alveoli occurs, resulting in an alveolar pulmonary edema. There are three main causes for the development of pulmonary edema:
In patients with a left-heart insufficiency, an increases hydrostatic capillary pressure and subsequent congestion in the lung blood vessels can lead to cardiogenic pulmonary edema.
In patients with nephritic syndrome or fluid overload of the body (e. g. by infusions), reduced plasma protein levels (hypo-albuminemia) can cause renal pulmonary edema.
In patients with lung infections (pneumoniae) or blood infection (sepsis) and in patients aspirating gastric contents, direct or indirect lung injuries can damage the epithelium and increase vascular permeability; the accompanying inflammatory processes enhance capillary permeability. The clinical man i-festations of the resulting toxic pulmonary edema is often acute respiratory distress syndrome (ARDS).

Several studies indicate that active salt transport by pulmonary epithelial cells drives osmotic water transport in the distal airspaces of the lung, a process known as lung liquid clearance (LLC, reviewed in (Matthay, M. A. et al. J Appl Physiol, 93(4):1533-41, 2002). Active fluid resorption can occur in all seg-ments of the pulmonary epithelium. However, since alveolar epithelial cells comprise 99% of the total airway surface, it is likely that the alveolar epithelium plays a predominant role, although the distal bron-chiolar epithelium may contribute (Matthay, M. A. et al, Proc. Am. Thorac. Soc., 2(3): 206-13, 2005).

Most experimental studies have attributed a primary role for a vectorial sodium transport in LLC. Sodium ions enter pulmonary epithelial cells on the apical side through the amiloride-sensitive sodium channel ENaC and are subsequently transported across the basolateral membrane by the Ouabain-inhibitable Na,K-ATPase (Matthay, M. A. et al. J Appl Physiol, 93(4):1533-41, 2002; Matthay, M. A. et al., Physiol Rev, 82(3):569-600, 2002.; Garty H. and Palmer L.G., Physiol Rev., 77(2), 359-96, 1997; Skou, J. C. and Esmann, M,. J Bioenerg.Biomembr., 24(3):249-61,1992). Water follows passively the generated osmotic force. There is evidence arising that also the glibenclamide-inhibitable chloride channel CFTR may contribute to LLC (Fang, X., et al., J.Gen. Physiol,119(2):199-207, 2002; Berthiaume, Y., et al., J Appl Physiol, 93(6):2207-13, 2002). This vectorial salt and water transport accounts for the ability of the human lung to remove water at the time of birth as well as in the mature lung when pathological conditions lead to the development of pulmonary edema. To date there is a limiting capac-ity of substances to treat pulmonary edema. Therefore it is an urgent need to identify new compounds that stimulate LLC.

Therefore, "pulmonary edema" or "lung edema" within the meaning of the present invention is to be understood as meaning lung edema of any origin, more specifically lung edema selected from the group comprising acute respiratory distress syndrome (ARDS) and acute lung injury (ALI) each in adults and children, of any cause, including but not limited to sepsis and septic shock syndrome and trauma; lung edema due to to low oncotic blood plasma pressure; cardiogenic lung edema due to, including but not limited to, the following causes congestive heart failure, myocardial infarction, pericardial tamponade, cardiac rhythm disorders, mitral stenosis and other heart valve disorders, acute left ventricular failure; infectious pneumonia; pulmonary edema due to inhaled toxins, endotoxemia, systemic intoxication by foreign substances, acute radiation pneumonitis, disseminated intravascular coagulopathy, immunological-hypersensitivity pneumonitis, acute pancreatitis; pulmonary edema following lung transplantation; high-altitude lung edema; neurogenic lung edema; lung edema during eclampsia/pre-eclampsia; in the course of pulmonary embolism; renal pulmonary edema and lung edema following cardiopulmonary bypass.

In an even more preferred embodiment of the present invention, the disease comprises one or more members selected from the group consisting of lung edema of any origin, more specifically lung edema selected from the group comprising acute respiratory distress syndrome (ARDS) and acute lung injury (ALI) each in adults and children, of any cause, including but not limited to sepsis and septic shock syndrome and trauma; lung edema due to to low oncotic blood plasma pressure; cardiogenic lung edema due to, including but not limited to, the following causes congestive heart failure, myocardial infarction, pericardial tamponade, cardiac rhythm disorders, mitral stenosis and other heart valve disorders, acute left ventricular failure; infectious pneumonia; pulmonary edema due to inhaled toxins, endotoxemia, systemic intoxication by foreign substances, acute radiation pneumonitis, disseminated intravascular coagulopathy, immunological-hypersensitivity pneumonitis, acute pancreatitis; pulmonary edema following lung transplantation; high-altitude lung edema; neurogenic lung edema; lung edema during eclampsia/pre-eclampsia; in the course of pulmonary embolism; renal pulmonary edema and lung edema following cardiopulmonary bypass.

More specifically, the peptides of the present invention can be used for the treatment of all phases of ARDS or ALI, in particular early to mid-phase, when no fibrotic reorganisation has occurred yet.

Accordingly, the phrase "treating pulmonary edema" refers to any intervention, therapy, condition, or alteration that reverses partially or fully the above mentioned mechanisms of liquid-transfer from the pulmonary vessels to the interstitial spaces and the alveolar lumen and reverses both hydrostatic and permeability edema.

In another aspect of present invention there is provided a pharmaceutical composition for inhalation comprising as a free combination (1) an effective amount of a pulmonary surfactant and optionally a pharmaceutically acceptable carrier and (2) an effective amount of one of the herein before mentioned peptides and optionally a pharmaceutically acceptable carrier.

In another aspect of present invention there is provided a pharmaceutical composition comprising an effective amount of a pulmonary surfactant and an effective amount of one of the herein before mentioned peptides for the treatment of ARDS, IRDS (idiopathic respiratory distress syndrome), ALI or lung edema.

In another aspect of present invention there is provided the use of a pharmaceutical composition comprising an effective amount of a pulmonary surfactant and an effective amount of one of the herein before mentioned peptides for the manufacture of a medicament for the treatment of a disease selected from the group consisting of ARDS, IRDS, ALI and lung edema.

In another aspect of present invention there is provided the use of a pharmaceutical composition comprising an effective amount of a pulmonary surfactant and an effective amount of one of the herein before mentioned peptides for the treatment of a disease selected from the group consisting of ARDS, IRDS, ALI and lung edema.

In another aspect of present invention there is provided a method for treating ARDS, IRDS, ALI or lung edema by administering to a patient in need thereof an effective amount of a pharmaceutical composition pharmaceutical composition comprising an effective amount of a pulmonary surfactant and an effective amount of one of the herein before mentioned peptides.

Pharmaceutical compositions of the present invention comprising a combination of a pulmonary surfactant and a peptide may have beneficial effects compared to pharmaceutical compositions comprising solely one of these active ingredients. Such a synergistic effect is described in WO2006013183. The synergistic effect of a novel peptide of the invention and a pulmonary surfactant on arterial blood oxygenation after repeated saline lung lavage in rats can be tested as described in WO2006013183.

Firstly, a synergistic benefit may be seen by administering a combination of a pulmonary surfactant and a peptide of present invention to a patient suffering from ARDS, IRDS, ALI or lung edema. For instance, it may be possible by using a combination of a pulmonary surfactant and a peptide of present invention to superiorly ameliorate oxygenation in a patient in need thereof suffering from ARDS, IRDS, ALI or lung edema.

Secondly, as a result of the improved oxygenation there may be provided a significantly improvement of patients body performance - compared to the use of a pulmonary surfactant alone.

Thirdly, because of the above-mentioned synergistic effect of a combination of present invention, the amount of the pulmonary surfactant may be significantly reduced when used in a combination with a peptide of present invention. As pulmonary surfactant are comparatively costly and peptides of present invention may be produced cost-saving by chemical synthesis, the cost of a treatment of a patient suffering from ARDS, IRDS, ALI or lung edema may be significantly reduced.

Fourthly, when using a combination product of present invention for the treatment of a patient suffering from ARDS, IRDS, ALI or lung edema, the frequency of ungratefulness related to the application of a pulmonary surfactant, for example, by instillation may also be reduced compared to the use of a pulmonary surfactant alone.

Fifthly, the use of a combination of a pulmonary surfactant and a peptide of present invention may significantly reduce the time patients with ARDS or IRDS have to be ventilated, and thus, it may possible by the administration of a combination of a pulmonary surfactant and a peptide of present invention to avoid side effects of ventilation, for example the risk of a nosocomial infection or pneumonia for the patients can be lowered compared to the use of a pulmonary surfactant alone.

Finally, it has been proven advantageous to prescribe combinations of single active ingredients in the form of a "patient pack" containing the whole course of treatment in a single package. This procedure is also applicable for the pharmaceutical compositions of present invention. Patient packs have an advantage over traditional prescriptions, where a pharmacist divides a patient's supply of a pharmaceutical from a bulk supply, in that the patient always has access to the package insert contained in the patient pack, normally missing in traditional prescriptions. The inclusion of a package insert has been shown to improve patient compliance with the physician's instructions and, therefore, lead generally to more successful treatment. It will be understood that the administration of a combination of present invention by means of a single patient pack, or patient packs of each component compound, and containing a package insert instructing the patient to the correct use of the invention is a desirable additional feature of the invention leading to an increased compliance of the patient compared to the administration of each single component.

### Examples

### Synthesis of peptides

The compounds according to the present invention can be prepared as described hereinafter and shown in the following reaction schemes, or as specified by way of example in the following examples or similarly or analogously thereto.

Peptides were prepared by fully automated solid-phase peptide synthesis using Fmoc/tBu-strategy and Fmoc-amino acid-TCP-polystyrene resin. The resin was distributed in 30 mg aliquots (15 mmol) to filter tubes, which were positioned in the format of a microtiter plate on valve blocks. Fmoc deprotections were carried out two times, 10 min. each, with 30 % piperidine in dimethylformamide (DMF) (300 ml). Nine washing steps were done with DMF (300 ml). Fmoc-amino acids (0.5 M) were dissolved with 1-hydroxybenzotriazole (HOBt) (0.5 M) in DMF. Diisopropylethylamine [3 M in NMP, 60 ml] was added to the reaction vessels. Coupling reagents diisopropylcarbodiimide [3 M in DMF, 50 ml, first coupling] or 1 H-benzotriazolium-1-[bis(dimethylamino)methylene]-5chloro-,tetrafluoroborate(1-),3-oxide [0.5 M in DMF, 200 ml, second coupling] and Fmoc-amino acids (first coupling 200 ml; second coupling 100 ml) were distributed to the reaction vessels. After 1 h coupling time, coupling reagents were filtered off and the resins were washed with DMF (1 x 200 ml) followed by a second coupling step (1 h). Amino acids were introduced using a sevenfold molar excess of the respective Fmoc-L-amino acid. After washing steps (4 x 400 ml) Fmoc deprotection was carried out two times, 10 min. each, with 30 % piperidine in DMF (300 ml). Nine washing steps were done with DMF (300 ml). The coupling process was repeated according to the length of the peptide.

The peptides were cleaved off the resin and side-chain deprotected with trifluoroacetic acid/phenol/ethanedithiol/thioanisole/water (95:2:1:1:1) (500 µl) Peptides were precipitated and washed three times with n-heptane/diethylether (1:1). The peptides were then lyophilized in tert.butyl alcohol/water (4:1).

Formation of intramolecular S-S bridges was carried out by oxidative cyclization by the action of iodine (0.1 mM solution of peptide in acetic acid/water (1:1, v/v) and 10 eq. lodine, 0.5 M in methanol). After 30 min 12 eq. ascorbic acid (1 M in H₂O) was added and the solvents were evaporated in vacuo.

The residue was dissolved in water and purified by preparative RP-HPLC. The product containing fractions were defined by investigation with analytical RP-HPLC mass spectrometry and evaporated. The residue was dissolved in acetic acid, diluted with tert.butyl alcohol (1:20) and lyophilised.

### Cell Culture

Calu-3 cells (ATCC, Rockville, MD) were grown in Earle's Modified Eagle Medium (MEM) with L Glutamine (PAA Laboratories, Pasching, Austria) supplemented with 10% fetal bovine serum (Sigma), 1 mM MEM sodium pyruvate (Invitrogen, Karlsruhe, Germany), 1 mM MEM nonessential amino acids (Invitrogen), 100 U/ml penicillin and 100 pglml streptomycin (Invitrogen) in 5% C02/95% air in a humidified atmosphere.

### TEER assay

Cells were seeded at a density of 1.5 x 10' cells/cm2 onto Transwell-Clear 24- well cell culture insert (Corning Costar, Bodenheim, Germany). By aspirating the apical fluid 48 h after cell seeding an air-liquid interface was created. The basolateral medium was changed every other day and monolayers were used for bioelectrical studies between day 7 and 10 post seeding. Formation of tight monolayers was tested by measuring the transepithelial electrical resistance (TEER) by using an EVOMX epithelial volthometer (World Precision Instruments (WPI), Sarasota, USA) in combination with chopstick STX2 electrodes.

For TEER assay measurements cell monolayers were transferred to an ENDOHM-6 electrode chamber (WPI) and bathed on both sides with Hank's balanced salt solution (HBSS) supplemented with 10 mM Hepes, pH 7.4. Once TEER reached steady state values, measurements were started. Recordings of TEER before and after addition of peptides (250 yM) to the apical and basolateral compartment were documented with an EVOMX epithelial Voltohmmeter connected to a Rec I I I recorder (Amersham Pharmacia, Uppsala, Sweden). Peak TEER values after the addition of the peptides were calculated as percent of the activity of the Tip peptide (= 100%).

For ion-substitution experiments HBSS is replaced by either of the following buffer solutions: (1) NaCl-buffer: 140 mM NaCl, 5.4 mM KCI, 1.25 mM CaCl2, 1 mM MgSO4, 10 mM HEPES, 5 mM glucose, (2) NMDG- and (3) Gluconate-buffer: NaCl is replaced by equimolar amounts of N-methyl-D-glucamine (NMDG) or Na+-gluconate, respectively, (4) CsCl-buffer: KCI is replaced by equimolar amounts of CsCl. The pH of all solutions is adjusted to 7.4.

For sodium titration experiments a dilution series of NaCl-buffer with NMDG-buffer is made to obtain the designated sodium concentration in the assay buffer.

| SEQ ID NO: | linear or circularized via disulfide bond | Amino acid sequence | Activity in TEER assay (c = 250 µM) (% of Tip activity) |
|---|---|---|---|
| 61 | circularized | CGQRETPEGAEAKPWYC | 100,0 |
| 2 | circularized | CGTKPIELGPDEPKAVC | 130,1 |
| 20 | linear | GGSPSQRETPEGAEAKPWYGGSPSQRET PEGAEAKPWYGGSPSQRETPEGAEAKPWY | 539,0 |
| 35 | circularized | CGQRESPEGAEAKPWYC | 120,9 |
| 39 | circularized | CGQRESPDGAEAKPWYC | 88,8 |
| 36 | circularized | CGQRESPEGADAKPWYC | 119,4 |
| 40 | circularized | CGQRESPDGADAKPWYC | 127,7 |
| 46 | circularized | CGQRETPDGAEAKPWYC | 130,4 |
| 43 | circularized | CGQRETPEGADAKPWYC | 139,8 |
| 45 | circularized | CGQRETPDGADAKPWYC | 172,4 |
| 42 | circularized | CGQRESPDGGDAKPWYC | 123,6 |
| 11 | linear | TPEGAEGGGGGTPEGAEGGGGGTPEGAE | 80,0 |
| 49 | circularized | CGTPEGAETPEGAETPEGAEGC | 139,9 |
| 50 | circularized | CGGGGGTPEGAEGGGGGTPEGAEGGGGGTPEGA EGC | 156,5 |
| 5 | linear | TPEGAETPEGAETPEGAETPEGAETPEGAE | 93,0 |
| 51 | circularized | CGTPEGAETPEGAETPEGAETPEGAETPEGAEGC | 146,6 |
| 52 | linear | SPEGGEGGGGGSPEGGEGGGGGSPEGGE | 87,1 |
| 53 | circularized | CGSPEGGEGGGGGSPEGGEGGGGGSPEGGEGC | 131,7 |
| 54 | linear | SPEGGDGGGGGSPEGGDGGGGGSPEGGD | 90,7 |
| 55 | circularized | CGSPEGGDGGGGGSPEGGDGGGGGSPEGGDGC | 146,0 |

### "Endotoxin induced lung injury in the isolated blood perfused flooded rat lung model"

In the "Endotoxin induced lung injury in the isolated blood perfused flooded rat lung model", which is an *ex vivo* permeability lung edema model, *E. coli*-LPS is instilled intratracheally and after approximately 5 hours the lung is isolated and placed in an artificial thorax chamber. Then, perfusion occurs with autologous blood. After an one hour stabilisation period, the test compound (in 1 ml saline) is instilled intratracheally. Lung parameters are measured over 2 hours. The primary readout parameters of this in vivo model are wet weight of lung and heart and P/V curves which are parameters of lung mechanics and hemodynamics.

### Fixed combination LUSUPULTIDE + novel peptide for intrabronchial instillation

9.8 g of 1,2-dipalmitoyl-3-sn-phosphatidylcholine, 4.2 g of 1-palmitoyl-2-oleoyl-3-sn-phosphatidyl-glycerolammonium, 0.7 g of palmitic acid, 0.36 g of calcium chloride and 0.28 g of r-SP-C (FF/I) are dissolved in 820 ml of 2-propanol/water (90:10) and spray-dried in a Büchi B 191 laboratory spray-dryer. Spray conditions: drying gas nitrogen, inlet temperature 110°C, outlet temperature 59-61°C. A fine powder is obtained which can be micronized. 180 mg of the novel peptide is dissolved in 180 ml 0.9% sodium chloride. The 15.34 g of the surfactant composition are added to this solution and suspended. For a single application in humans 1 ml/kg body weight of this suspension can be instilled intrabronchially guided by a bronchoscope.

### Fixed combination LUSUPULTIDE + novel peptide for intratracheal nebulization

9.8 g of 1,2-dipalmitoyl-3-sn-phosphatidylcholine, 4.2 g of 1-palmitoyl-2-oleoyl-3-sn-phosphatidyl-glycerolammonium, 0.7 g of palmitic acid, 0.36 g of calcium chloride and 0.28 g of r-SP-C (FF/I) are spray-dried as described in the foregoing example. 360 mg of the novel peptide is dissolved in 180 ml 0.9% sodium chloride. The 15.34 g of the surfactant composition are added to this solution and suspended. For a single application in humans 1 ml/kg body weight of this suspension can be nebulized by an Aeroneb Pro aerosol generator (Aerogen Inc.,Stierlin Court, CA).

### Free combination of BERACTANT for intratracheal instillation + novel peptide for aerosol administration

For a single application in humans commercially available BERACTANT (Survanta®) is administered intratracheally 100 mg/kg as a suspension in 0.9% sodium chloride containing 25 mg phospholipids per mL (consisting of 11.0 - 15.5 mg/ml disaturated phosphatidycholine, 0.5 - 1.75 mg/ml triglycerides, 1.4 - 3.5 mg/ml free fatty acids, and less than 1.0 mg/ml protein). This application is combined with one or several administrations of the novel peptide. Therefore, 2 mg/ml of the novel peptide is dissolved in 0.9% sodium chloride. For a single application in humans 1 ml/kg body weight of this suspension can be nebulized by an Aeroneb Pro aerosol generator (Aerogen Inc.,Stierlin Court, CA).

### Free combination PORACTANT ALPHA for intratracheal instillation + TIP-peptide for intratracheal instillation

For a single application in humans commercially available PORACTANT ALPHA (Curosurf®) is administered intratracheally 100-200 mg/kg. Composition per ml of suspension: phospholipid fraction from porcine lung 80 mg/ml, equivalent to about 74 mg/ml of total phospholipids and 0.9 mg/ml of low molecular weight hydrophobic proteins. This application is combined with one or several timed intratracheal administrations of 1 mg/kg of the novel peptide dissolved in 1 mg/ml 0.9% sodium chloride.

### Influence of combined administration of a novel peptide and LUSUPULTIDE on arterial blood oxygenation after repeated saline lung lavage in rats

Male Wistar rats (230-280 g) are anaesthetized, catheterized to withdraw arterial blood, and ventilated with pure oxygen (=> Pa0₂ ~ 500 - 550 mmHg). 30 min later lungs are lavaged 5-9 times with NaCl 0.9% (=> PaO₂ ~ 50 - 100 mmHg). After 60 min NaCl 0.9%, LUSUPULTIDE 12.5 mgPL/kg, novel peptide (500µg/animal), or LUSUPULTIDE (12.5 mgPL/kg) in combination with novel peptide (500 µg/animal) is administered intratracheally (administration volume 1.2 ml, solvent NaCl 0.9%). Arterial blood oxygenation (PaO₂) is determined every 30 min up to 150 min after drug administration (t = 210 min).

## Claims

1. Peptide having a sequence of 14 to 100 contiguous amino acids comprising the sequence TKPIELGPDEPKAV wherein one, two or three conservative amino acid substitutions are allowed, which peptide inhibits or reduces the accumulation of excess fluid in tissues.

2. Peptide according to claim 1, wherein the peptide consists of the sequence TKPIELGPDEPKAV.

3. Peptide according to claim 1, wherein the peptide consists of the sequence CGTKPIELGPDEPKAVC.

4. Peptide having a sequence of 18 to 94 contiguous amino acids comprising the hexameric sequence TPEGAE at least three times, which peptide inhibits or reduces the accumulation of excess fluid in tissues.

5. Peptide having a sequence of 42 to 94 contiguous amino acids comprising the sequence QRETPEGAEAKPWY at least three times, which peptide inhibits or reduces the accumulation of excess fluid in tissues.

6. Peptide having a sequence of 6 to 100 contiguous amino acids comprising the hexameric sequence X₁PX₂GX₃X₄ at least one time, wherein X₁ is selected from S, T and L, wherein X₂ is selected from D, E, Q and R, wherein X₃ is selected from A, S, T, E and G, and wherein X₄ is selected from D, E and K, with the proviso that the combination of amino acids X₁, X₂, and X₄ is not T, E, E, which peptide inhibits or reduces the accumulation of excess fluid in tissues.

7. Peptide according to claim 6, wherein the hexameric sequence X₁PX₂GX₃X₄ is selected from the group of sequences consisting of SPEGAE; SPEGGE; SPEGAD; SPEGGD; SPDGAE; SPDGAD; SPDGGD; SPDGGE; TPDGAE; TPDGAD; TPDGGE; TPDGGD; TPEGAD; and TPEGGD.

8. Peptide having a sequence of 14 to 100 contiguous amino acids comprising the sequence QREX₁PX₂GX₃X₄AKPWY at least one time, wherein X₁ is selected from S, T and L, wherein X₂ is selected from D, E, Q and R, wherein X₃ is selected from A, S, T, E and G, and wherein X₄ is selected from D, E and K, with the proviso that the combination of amino acids X₁, X₂, and X₄ is not T, E, E, which peptide inhibits or reduces the accumulation of excess fluid in tissues.

9. Peptide according to claim 8, wherein the sequence QREX₁PX₂GX₃X₄AKPWY is selected from the group of sequences consisting of SEQ ID NOs: 62, 63, 64, 65, 66, 67, 68, 69, 70, 71, 72, 73, 74, and 75.

10. Peptide according to any one of claims 6 and 7, wherein the hexameric sequence X₁PX₂GX₃X₄ is present in the peptide from two to eight times.

11. Peptide according to any one of claim 10, wherein the hexameric sequence X₁PX₂GX₃X₄ is present from three to five times.

12. Peptide according to any one of claims 6, 7, 10, 11, wherein the hexameric sequence X₁PX₂GX₃X₄ is present in the peptide as consecutive sequences.

13. Peptide according to any one of claims 6, 7, 10, 11, wherein the hexameric sequence X₁PX₂GX₃X₄ is separated by a linker sequence consisting of two to ten amino acids.

14. Peptide according to any one of claims 8 and 9, wherein the sequence QREX₁PX₂GX₃X₄AKPWY is present in the peptide from two to eight times.

15. Peptide according to any one of claim 14, wherein the sequence QREX₁PX₂GX₃X₄AKPWY is present from three to five times.

16. Peptide according to any one of claims 8, 9, 14, 15, wherein the sequence QREX₁PX₂GX₃X₄AKPWY is present in the peptide as consecutive sequences.

17. Peptide according to any one of claims 8, 9, 14, 15, wherein the sequence QREX₁PX₂GX₃X₄AKPWY is separated by a linker sequence consisting of two to ten amino acids.

18. Peptide according to any one of claims 13 and 17, wherein the linker sequence consists of five amino acids.

19. Peptide according to any one of claims 13, 17, 18, wherein the linker sequence consists of small amino acids selected from the group consisting of G, A, S, C, and P.

20. Peptide according to any one of claims 13, 17, 18, wherein the linker sequence is selected from the group consisting of the amino acid sequences GGGGG, GGCGG and GGSPS.

21. Peptide having an amino acid sequence selected from the group consisting of SEQ ID NOs: 3, 4, 5, 6, 7, 8, 9, 10, 11, 12, 13, 14, 19, 20, 35, 36, 37, 38, 39, 40, 41, 42, 43, 44, 45, 46, 47, 48, 49, 50, 51, 52, 53, 54, 55, 56, 57, 58, 59, 60, 62, 63, 64, 65, 66, 67, 68, 69, 70, 71, 72, 73, 74, and 75.

22. Peptide according to any one of claims 1 to 21, wherein the peptide is linear.

23. Peptide according to any one of claims 1 to 21, wherein the peptide is circularized.

24. Peptide according to claim 23, wherein the peptide is circularized via an amide bond of the peptide backbone.

25. Peptide according to claim 23, wherein the peptide is circularized via the side chain of C amino acid residues at the N- and C-terminus of the peptide forming a disulfide bond.

26. Peptide according to claim 23, wherein intramolecular disulfide bonds are formed within the peptide sequence via the side chains of C amino acid residues.

27. Peptide according to any one of claims 1 to 26, wherein the peptide is a synthetic peptide.

28. Peptide according to any one of claims 1 to 26, wherein the peptide is produced recombinantly.

29. Peptide mixture containing at least one peptide according to any one of claims 1 to 28.

30. A peptide or a peptide mixture according to any one of claims 1 to 29 or a pharmaceutically acceptable salt thereof for use in the treatment and/or prophylaxis of diseases.

31. A pharmaceutical composition comprising one or more peptides according to any one of claims 1 to 28, or a pharmaceutically acceptable salt thereof together with one or more pharmaceutically acceptable auxiliaries and/or excipients.

32. Use of a peptide according to any one of claims 1 to 28 or a pharmaceutically acceptable salt thereof in the manufacture of a pharmaceutical composition for the treatment and or prevention of a disease, in which inhibition or reduction of excess fluid accumulation in tissues is beneficial.

33. The use according to claim 32 wherein said disease is edema.

34. The use according to any on of claims 32 or 33 wherein said disease comprises one or more members selected from the group consisting of lung edema of any origin, comprising acute respiratory distress syndrome (ARDS) and acute lung injury (ALI) each in adults and children, of any cause, including but not limited to sepsis and septic shock syndrome and trauma; lung edema due to low oncotic blood plasma pressure; cardiogenic lung edema due to, including but not limited to, the following causes congestive heart failure, myocardial infarction, pericardial tamponade, cardiac rhythm disorders, mitral stenosis and other heart valve disorders, acute left ventricular failure; infectious pneum o-nia; pulmonary edema due to inhaled toxins, endotoxemia, systemic intoxication by foreign substances, acute radiation pneumonitis, disseminated intravascular coagulopathy, immunological-hypersensitivity pneumonitis, acute pancreatitis; pulmonary edema following lung transplantation; high-altitude lung edema; neurogenic lung edema; lung edema during eclampsia/pre-eclampsia; in the course of pulmonary embolism; and lung edema following cardiopulmonary bypass.

35. Pharmaceutical composition comprising an effective amount of a pulmonary surfactant and an effective amount of a peptide or a peptide mixture according to claims 1 to 29.

36. Pharmaceutical composition according to claim 35 comprising as a fixed combination an effective amount of a pulmonary surfactant and an effective amount of a peptide or a peptide mixture according to claims 1 to 29, and optionally a pharmaceutically acceptable carrier.

37. Pharmaceutical composition according to any one of claims 35 and 36, which is a powder formulation.

38. Pharmaceutical composition according to claim 35 and 36, which is a liquid formulation.

39. Pharmaceutical composition according to any one of claims 35 to 38, wherein the pulmonary surfactant is selected from the group consisting of PORACTANT ALFA, BERACTANT, BOVACTANT, COLFOSCERIL PALMITATE, SURFACTANT-TA, CALFACTANT, PUMACTANT, LUSUPULTIDE OR SINAPULTIDE.

40. Pharmaceutical composition according to any one of claims 35 to 39 for the treatment of ARDS, IRDS, ALI or lung edema.

41. Use of a pharmaceutical composition according to any one of claims 35 to 39 for the manufacture of a medicament for the treatment of a disease selected from the group consisting of ARDS, IRDS, ALI and lung edema.


SEQUENCE LISTING

<110>  ALTANA Pharma AG

<120>  NOVEL PEPTIDES AND THEIR USE FOR THE TREATMENT OF EDEMA

<130>  1476EPORD01

<160>  76

<170>  PatentIn version 3.3

<210>  1
<211>  6
<212>  PRT
<213>  Homo sapiens

<400>  1

Thr Pro Glu Gly Ala Glu
1               5


<210>  2
<211>  17
<212>  PRT
<213>  Artificial

<220>
<223>  anti-edema peptide

<400>  2

Cys Gly Thr Lys Pro Ile Glu Leu Gly Pro Asp Glu Pro Lys Ala Val
1               5                   10                  15


Cys



<210>  3
<211>  18
<212>  PRT
<213>  Artificial

<220>
<223>  anti-edema peptide

<400>  3

Thr Pro Glu Gly Ala Glu Thr Pro Glu Gly Ala Glu Thr Pro Glu Gly
1               5                   10                  15


Ala Glu

<210>  4
<211>  24
<212>  PRT
<213>  Artificial

<220>
<223>  anti-edema peptide

<400>  4

Thr Pro Glu Gly Ala Glu Thr Pro Glu Gly Ala Glu Thr Pro Glu Gly
1               5                   10                  15

Ala Glu Thr Pro Glu Gly Ala Glu
            20

<210>  5
<211>  30
<212>  PRT
<213>  Artificial

<220>
<223>  anti-edema peptide

<400>  5

Thr Pro Glu Gly Ala Glu Thr Pro Glu Gly Ala Glu Thr Pro Glu Gly
1               5                   10                  15

Ala Glu Thr Pro Glu Gly Ala Glu Thr Pro Glu Gly Ala Glu
            20                  25                  30

<210>  6
<211>  20
<212>  PRT
<213>  Artificial

<220>
<223>  anti-edema peptide

<400>  6

Cys Thr Pro Glu Gly Ala Glu Thr Pro Glu Gly Ala Glu Thr Pro Glu
1               5                   10                  15

Gly Ala Glu Cys
            20

<210>  7
<211>  26
<212>  PRT
<213>  Artificial

<220>
<223>  anti-edema peptide

<400>  7

Cys Thr Pro Glu Gly Ala Glu Thr Pro Glu Gly Ala Glu Thr Pro Glu
1               5                   10                  15

Gly Ala Glu Thr Pro Glu Gly Ala Glu Cys
            20                  25

<210>  8
<211>  32
<212>  PRT
<213>  Artificial

<220>
<223>  anti-edema peptide

<400>  8

Cys Thr Pro Glu Gly Ala Glu Thr Pro Glu Gly Ala Glu Thr Pro Glu
1               5                   10                  15

Gly Ala Glu Thr Pro Glu Gly Ala Glu Thr Pro Glu Gly Ala Glu Cys
            20                  25                  30

<210>  9
<211>  24
<212>  PRT
<213>  Artificial

<220>
<223>  anti-edema peptide

<400>  9

Thr Pro Glu Gly Ala Glu Gly Gly Gly Thr Pro Glu Gly Ala Glu Gly
1               5                   10                  15

Gly Gly Thr Pro Glu Gly Ala Glu
            20

<210>  10
<211>  26
<212>  PRT
<213>  Artificial

<220>
<223>  anti-edema peptide

<400>  10

Thr Pro Glu Gly Ala Glu Gly Gly Gly Gly Thr Pro Glu Gly Ala Glu
1               5                   10                  15

Gly Gly Gly Gly Thr Pro Glu Gly Ala Glu

20                  25


<210>  11
<211>  28
<212>  PRT
<213>  Artificial

<220>
<223>  anti-edema peptide

<400>  11

Thr Pro Glu Gly Ala Glu Gly Gly Gly Gly Gly Thr Pro Glu Gly Ala
1               5                   10                  15


Glu Gly Gly Gly Gly Gly Thr Pro Glu Gly Ala Glu
            20                  25


<210>  12
<211>  26
<212>  PRT
<213>  Artificial

<220>
<223>  anti-edema peptide

<400>  12

Cys Thr Pro Glu Gly Ala Glu Gly Gly Gly Thr Pro Glu Gly Ala Glu
1               5                   10                  15


Gly Gly Gly Thr Pro Glu Gly Ala Glu Cys
            20                  25


<210>  13
<211>  28
<212>  PRT
<213>  Artificial

<220>
<223>  anti-edema peptide

<400>  13

Cys Thr Pro Glu Gly Ala Glu Gly Gly Gly Gly Thr Pro Glu Gly Ala
1               5                   10                  15


Glu Gly Gly Gly Gly Thr Pro Glu Gly Ala Glu Cys
            20                  25


<210>  14
<211>  30
<212>  PRT
<213>  Artificial

<220>
<223>  anti-edema peptide

<400>  14

Cys Thr Pro Glu Gly Ala Glu Gly Gly Gly Gly Gly Thr Pro Glu Gly
1               5                   10                  15

Ala Glu Gly Gly Gly Gly Gly Thr Pro Glu Gly Ala Glu Cys
            20                  25                  30

<210>  15
<211>  5
<212>  PRT
<213>  Artificial

<220>
<223>  linker sequence

<400>  15

Gly Gly Gly Gly Gly
1               5

<210>  16
<211>  5
<212>  PRT
<213>  Artificial

<220>
<223>  linker sequence

<400>  16

Gly Gly Cys Gly Gly
1               5

<210>  17
<211>  5
<212>  PRT
<213>  Artificial

<220>
<223>  linker sequence

<400>  17

Gly Gly Ser Pro Ser
1               5

<210>  18
<211>  14
<212>  PRT
<213>  Homo sapiens

<400>  18

Gln Arg Glu Thr Pro Glu Gly Ala Glu Ala Lys Pro Trp Tyr
1               5                   10

<210>  19
<211>  42
<212>  PRT
<213>  Artificial

<220>
<223>  anti-edema peptide

<400>  19

Gln Arg Glu Thr Pro Glu Gly Ala Glu Ala Lys Pro Trp Tyr Gln Arg
1               5                   10                  15

Glu Thr Pro Glu Gly Ala Glu Ala Lys Pro Trp Tyr Gln Arg Glu Thr
            20                  25                  30

Pro Glu Gly Ala Glu Ala Lys Pro Trp Tyr
        35                  40

<210>  20
<211>  57
<212>  PRT
<213>  Artificial

<220>
<223>  anti-edema peptide

<400>  20

Gly Gly Ser Pro Ser Gln Arg Glu Thr Pro Glu Gly Ala Glu Ala Lys
1               5                   10                  15

Pro Trp Tyr Gly Gly Ser Pro Ser Gln Arg Glu Thr Pro Glu Gly Ala
            20                  25                  30

Glu Ala Lys Pro Trp Tyr Gly Gly Ser Pro Ser Gln Arg Glu Thr Pro
        35                  40                  45

Glu Gly Ala Glu Ala Lys Pro Trp Tyr
    50                  55

<210>  21
<211>  6
<212>  PRT
<213>  Artificial

<220>
<223>  anti-edema peptide

<400>  21

Ser Pro Glu Gly Ala Glu
1               5


<210>  22
<211>  6
<212>  PRT
<213>  Artificial

<220>
<223>  anti-edema peptide

<400>  22

Ser Pro Glu Gly Gly Glu
1               5


<210>  23
<211>  6
<212>  PRT
<213>  Artificial

<220>
<223>  anti-edema peptide

<400>  23

Ser Pro Glu Gly Ala Asp
1               5


<210>  24
<211>  6
<212>  PRT
<213>  Artificial

<220>
<223>  anti-edema peptide

<400>  24

Ser Pro Glu Gly Gly Asp
1               5


<210>  25
<211>  6
<212>  PRT
<213>  Artificial

<220>
<223>  anti-edema peptide

<400>  25

Ser Pro Asp Gly Ala Glu
1               5


<210>  26
<211>  6

<212>  PRT
<213>  Artificial

<220>
<223>  anti-edema peptide

<400>  26

Ser Pro Asp Gly Ala Asp
1               5


<210>  27
<211>  6
<212>  PRT
<213>  Artificial

<220>
<223>  anti-edema peptide

<400>  27

Ser Pro Asp Gly Gly Asp
1               5


<210>  28
<211>  6
<212>  PRT
<213>  Artificial

<220>
<223>  anti-edema peptide

<400>  28

Ser Pro Asp Gly Gly Glu
1               5


<210>  29
<211>  6
<212>  PRT
<213>  Artificial

<220>
<223>  anti-edema peptide

<400>  29

Thr Pro Asp Gly Ala Glu
1               5


<210>  30
<211>  6
<212>  PRT
<213>  Artificial

<220>
<223>  anti-edema peptide

<400>  30

Thr Pro Asp Gly Ala Asp
1               5


<210>  31
<211>  6
<212>  PRT
<213>  Artificial

<220>
<223>  anti-edema peptide

<400>  31

Thr Pro Asp Gly Gly Glu
1               5


<210>  32
<211>  6
<212>  PRT
<213>  Artificial

<220>
<223>  anti-edema peptide

<400>  32

Thr Pro Asp Gly Gly Asp
1               5


<210>  33
<211>  6
<212>  PRT
<213>  Artificial

<220>
<223>  anti-edema peptide

<400>  33

Thr Pro Glu Gly Ala Asp
1               5


<210>  34
<211>  6
<212>  PRT
<213>  Artificial

<220>
<223>  anti-edema peptide

<400>  34

Thr Pro Glu Gly Gly Asp
1               5

<210>  35
<211>  17
<212>  PRT
<213>  Artificial

<220>
<223>  anti-edema peptide

<400>  35

Cys Gly Gln Arg Glu Ser Pro Glu Gly Ala Glu Ala Lys Pro Trp Tyr
1               5                   10                  15

Cys

<210>  36
<211>  17
<212>  PRT
<213>  Artificial

<220>
<223>  anti-edema peptide

<400>  36

Cys Gly Gln Arg Glu Ser Pro Glu Gly Ala Asp Ala Lys Pro Trp Tyr
1               5                   10                  15

Cys

<210>  37
<211>  17
<212>  PRT
<213>  Artificial

<220>
<223>  anti-edema peptide

<400>  37

Cys Gly Gln Arg Glu Ser Pro Glu Gly Gly Glu Ala Lys Pro Trp Tyr
1               5                   10                  15

Cys

<210>  38
<211>  17
<212>  PRT
<213>  Artificial

<220>
<223>  anti-edema peptide

<400>  38

Cys Gly Gln Arg Glu Ser Pro Glu Gly Gly Asp Ala Lys Pro Trp Tyr
1               5                   10                  15

Cys

<210>  39
<211>  17
<212>  PRT
<213>  Artificial

<220>
<223>  anti-edema peptide

<400>  39

Cys Gly Gln Arg Glu Ser Pro Asp Gly Ala Glu Ala Lys Pro Trp Tyr
1               5                   10                  15

Cys

<210>  40
<211>  17
<212>  PRT
<213>  Artificial

<220>
<223>  anti-edema peptide

<400>  40

Cys Gly Gln Arg Glu Ser Pro Asp Gly Ala Asp Ala Lys Pro Trp Tyr
1               5                   10                  15

Cys

<210>  41
<211>  17
<212>  PRT
<213>  Artificial

<220>
<223>  anti-edema peptide

<400>  41

Cys Gly Gln Arg Glu Ser Pro Asp Gly Gly Glu Ala Lys Pro Trp Tyr
1               5                   10                  15

Cys

<210>  42
<211>  17
<212>  PRT
<213>  Artificial

<220>
<223>  anti-edema peptide

<400>  42

Cys Gly Gln Arg Glu Ser Pro Asp Gly Gly Asp Ala Lys Pro Trp Tyr
1               5                   10                  15

Cys

<210>  43
<211>  17
<212>  PRT
<213>  Artificial

<220>
<223>  anti-edema peptide

<400>  43

Cys Gly Gln Arg Glu Thr Pro Glu Gly Ala Asp Ala Lys Pro Trp Tyr
1               5                   10                  15

Cys

<210>  44
<211>  17
<212>  PRT
<213>  Artificial

<220>
<223>  anti-edema peptide

<400>  44

Cys Gly Gln Arg Glu Thr Pro Glu Gly Gly Asp Ala Lys Pro Trp Tyr
1               5                   10                  15

Cys

<210>  45
<211>  17
<212>  PRT
<213>  Artificial

<220>

<223>  anti-edema peptide

<400>  45

Cys Gly Gln Arg Glu Thr Pro Asp Gly Ala Asp Ala Lys Pro Trp Tyr
1               5                   10                  15

Cys

<210>  46
<211>  17
<212>  PRT
<213>  Artificial

<220>
<223>  anti-edema peptide

<400>  46

Cys Gly Gln Arg Glu Thr Pro Asp Gly Ala Glu Ala Lys Pro Trp Tyr
1               5                   10                  15

Cys

<210>  47
<211>  17
<212>  PRT
<213>  Artificial

<220>
<223>  anti-edema peptide

<400>  47

Cys Gly Gln Arg Glu Thr Pro Asp Gly Gly Asp Ala Lys Pro Trp Tyr
1               5                   10                  15

Cys

<210>  48
<211>  17
<212>  PRT
<213>  Artificial

<220>
<223>  anti-edema peptide

<400>  48

Cys Gly Gln Arg Glu Thr Pro Asp Gly Gly Glu Ala Lys Pro Trp Tyr
1               5                   10                  15

Cys

<210> 49
<211> 22
<212> PRT
<213> Artificial

<220>
<223> anti-edema peptide

<400> 49

Cys Gly Thr Pro Glu Gly Ala Glu Thr Pro Glu Gly Ala Glu Thr Pro
1               5                   10                  15

Glu Gly Ala Glu Gly Cys
            20

<210> 50
<211> 36
<212> PRT
<213> Artificial

<220>
<223> anti-edema peptide

<400> 50

Cys Gly Gly Gly Gly Gly Thr Pro Glu Gly Ala Glu Gly Gly Gly Gly
1               5                   10                  15

Gly Thr Pro Glu Gly Ala Glu Gly Gly Gly Gly Gly Thr Pro Glu Gly
            20                  25                  30

Ala Glu Gly Cys
        35

<210> 51
<211> 34
<212> PRT
<213> Artificial

<220>
<223> anti-edema peptide

<400> 51

Cys Gly Thr Pro Glu Gly Ala Glu Thr Pro Glu Gly Ala Glu Thr Pro
1               5                   10                  15

Glu Gly Ala Glu Thr Pro Glu Gly Ala Glu Thr Pro Glu Gly Ala Glu
            20                  25                  30

Gly Cys

<210> 52
<211> 28
<212> PRT
<213> Artificial

<220>
<223> anti-edema peptide

<400> 52

Ser Pro Glu Gly Gly Glu Gly Gly Gly Gly Gly Ser Pro Glu Gly Gly
1               5                   10                  15

Glu Gly Gly Gly Gly Gly Ser Pro Glu Gly Gly Glu
            20                  25

<210> 53
<211> 32
<212> PRT
<213> Artificial

<220>
<223> anti-edema peptide

<400> 53

Cys Gly Ser Pro Glu Gly Gly Glu Gly Gly Gly Gly Gly Ser Pro Glu
1               5                   10                  15

Gly Gly Glu Gly Gly Gly Gly Gly Ser Pro Glu Gly Gly Glu Gly Cys
            20                  25                  30

<210> 54
<211> 28
<212> PRT
<213> Artificial

<220>
<223> anti-edema peptide

<400> 54

Ser Pro Glu Gly Gly Asp Gly Gly Gly Gly Gly Ser Pro Glu Gly Gly
1               5                   10                  15

Asp Gly Gly Gly Gly Gly Ser Pro Glu Gly Gly Asp
            20                  25

<210> 55
<211> 32
<212> PRT

<213>  Artificial

<220>
<223>  anti-edema peptide

<400>  55

Cys Gly Ser Pro Glu Gly Gly Asp Gly Gly Gly Gly Gly Ser Pro Glu
1               5                   10                  15

Gly Gly Asp Gly Gly Gly Gly Gly Ser Pro Glu Gly Gly Asp Gly Cys
            20                  25                  30

<210>  56
<211>  22
<212>  PRT
<213>  Artificial

<220>
<223>  anti-edema peptide

<400>  56

Cys Gly Ser Pro Glu Gly Gly Glu Ser Pro Glu Gly Gly Glu Ser Pro
1               5                   10                  15

Glu Gly Gly Glu Gly Cys
            20

<210>  57
<211>  22
<212>  PRT
<213>  Artificial

<220>
<223>  anti-edema peptide

<400>  57

Cys Gly Ser Pro Asp Gly Gly Asp Ser Pro Asp Gly Gly Asp Ser Pro
1               5                   10                  15

Asp Gly Gly Asp Gly Cys
            20

<210>  58
<211>  28
<212>  PRT
<213>  Artificial

<220>
<223>  anti-edema peptide

<400>  58

Ser Pro Asp Gly Gly Asp Gly Gly Gly Gly Gly Ser Pro Asp Gly Gly

1               5                   10                  15

Asp Gly Gly Gly Gly Gly Ser Pro Asp Gly Gly Asp
            20                  25

<210>  59
<211>  32
<212>  PRT
<213>  Artificial

<220>
<223>  anti-edema peptide

<400>  59

Cys Gly Ser Pro Asp Gly Gly Asp Gly Gly Gly Gly Gly Ser Pro Asp
1               5                   10                  15

Gly Gly Asp Gly Gly Gly Gly Gly Ser Pro Asp Gly Gly Asp Gly Cys
            20                  25                  30

<210>  60
<211>  22
<212>  PRT
<213>  Artificial

<220>
<223>  anti-edema peptide

<400>  60

Cys Gly Ser Pro Glu Gly Gly Asp Ser Pro Glu Gly Gly Asp Ser Pro
1               5                   10                  15

Glu Gly Gly Asp Gly Cys
            20

<210>  61
<211>  17
<212>  PRT
<213>  Artificial

<220>
<223>  anti-edema peptide

<400>  61

Cys Gly Gln Arg Glu Thr Pro Glu Gly Ala Glu Ala Lys Pro Trp Tyr
1               5                   10                  15

Cys

<210>  62

<211>  14
<212>  PRT
<213>  Artificial

<220>
<223>  Synthetic construct

<400>  62

Gln Arg Glu Ser Pro Glu Gly Ala Glu Ala Lys Pro Trp Tyr
1               5                   10


<210>  63
<211>  14
<212>  PRT
<213>  Artificial

<220>
<223>  anti-edema peptide

<400>  63

Gln Arg Glu Ser Pro Glu Gly Ala Asp Ala Lys Pro Trp Tyr
1               5                   10


<210>  64
<211>  14
<212>  PRT
<213>  Artificial

<220>
<223>  anti-edema peptide

<400>  64

Gln Arg Glu Ser Pro Glu Gly Gly Glu Ala Lys Pro Trp Tyr
1               5                   10


<210>  65
<211>  14
<212>  PRT
<213>  Artificial

<220>
<223>  anti-edema peptide

<400>  65

Gln Arg Glu Ser Pro Glu Gly Gly Asp Ala Lys Pro Trp Tyr
1               5                   10


<210>  66
<211>  14
<212>  PRT
<213>  Artificial

<220>
<223>  anti-edema peptide

<400>  66

Gln Arg Glu Ser Pro Asp Gly Ala Glu Ala Lys Pro Trp Tyr
1               5                   10


<210>  67
<211>  14
<212>  PRT
<213>  Artificial

<220>
<223>  anti-edema peptide

<400>  67

Gln Arg Glu Ser Pro Asp Gly Ala Asp Ala Lys Pro Trp Tyr
1               5                   10


<210>  68
<211>  14
<212>  PRT
<213>  Artificial

<220>
<223>  anti-edema peptide

<400>  68

Gln Arg Glu Ser Pro Asp Gly Gly Glu Ala Lys Pro Trp Tyr
1               5                   10


<210>  69
<211>  14
<212>  PRT
<213>  Artificial

<220>
<223>  anti-edema peptide

<400>  69

Gln Arg Glu Ser Pro Asp Gly Gly Asp Ala Lys Pro Trp Tyr
1               5                   10


<210>  70
<211>  14
<212>  PRT
<213>  Artificial

<220>
<223>  anti-edema peptide

<400>  70

Gln Arg Glu Thr Pro Glu Gly Ala Asp Ala Lys Pro Trp Tyr
1               5                   10

<210>  71
<211>  14
<212>  PRT
<213>  Artificial

<220>
<223>  anti-edema peptide

<400>  71

Gln Arg Glu Thr Pro Glu Gly Gly Asp Ala Lys Pro Trp Tyr
1               5                   10


<210>  72
<211>  14
<212>  PRT
<213>  Artificial

<220>
<223>  anti-edema peptide

<400>  72

Gln Arg Glu Thr Pro Asp Gly Ala Asp Ala Lys Pro Trp Tyr
1               5                   10


<210>  73
<211>  14
<212>  PRT
<213>  Artificial

<220>
<223>  anti-edema peptide

<400>  73

Gln Arg Glu Thr Pro Asp Gly Ala Glu Ala Lys Pro Trp Tyr
1               5                   10


<210>  74
<211>  14
<212>  PRT
<213>  Artificial

<220>
<223>  anti-edema peptide

<400>  74

Gln Arg Glu Thr Pro Asp Gly Gly Asp Ala Lys Pro Trp Tyr
1               5                   10


<210>  75
<211>  14
<212>  PRT
<213>  Artificial

<220>
<223>  anti-edema peptide

<400>  75

Gln Arg Glu Thr Pro Asp Gly Gly Glu Ala Lys Pro Trp Tyr
1               5                   10


<210>  76
<211>  14
<212>  PRT
<213>  Artificial

<220>
<223>  anti-edema peptide

<400>  76

Thr Lys Pro Ile Glu Leu Gly Pro Asp Glu Pro Lys Ala Val
1               5                   10